# EUROPEAN PATENT SPECIFICATION

(11) **EP 1 641 926 B1**
(45) Date of publication and mention of the grant of the patent: **16.03.2011**
(21) Application number: 04740883.6
(22) Date of filing: 08.07.2004
(51) Int. Cl.: A61K 39/085, A61P 37/06, A61K 38/16, A61K 48/00

(54) **THERAPEUTIC USE OF LPI, A STAPHYLOCOCCAL LECTIN PATHWAY INHIBITOR IN INFLAMMATORY DISEASES**
THERAPEUTISCHE VERWENDUNG VON LPI, EINEM INHIBITOR DES LECTINWEGS IN STAPHYLOKOKKEN BEI ENTZÜNDUNGSKRANKHEITEN
UTILISATION THERAPEUTIQUE D'INHIBITEUR DE LA VOIE DE LA LECTINE, INHIBITEUR DE LA VOIE DE LA LECTINE D'ORIGINE STAPHYLOCOCCIQUE DANS DES MALADIES INFLAMMATOIRES

(30) Priority: 08.07.2003 EP 03077138
(43) Date of publication of application: 05.04.2006
(73) Proprietor: UMC Utrecht Holding B.V., 3584 CM Utrecht (NL)
(72) Inventor: VAN WAMEL, Willem, Jan, Bastiaan, NL-3515 VK Utrecht (NL); ROOIJAKKERS, Suzan, Huberdina, Maria, NL-3512 PL Utrecht (NL); VAN KESSEL, Cornelis, Petrus, Maria, NL-3981 EX Bunnik (NL); VAN STRIJP, Johannes, Antonius, Gerardus, NL-3984 NV Odijk (NL)
(74) Representative: Van Someren, Petronella F. H. M.
(86) International application number: PCT/EP2004/007606
(87) International publication number: WO 2005/005630

(56) References cited:
- EP-A- 0 786 519
- WO-A-00/02913
- WO-A-94/06830
- WO-A-2004/087746
- DATABASE UniProt [Online] 1 December 2001 (2001-12-01), "Hypothetical protein." XP002322488 retrieved from EBI accession no. UNIPROT:Q931M7 Database accession no. Q931M7_STAAM & DATABASE UniProt [Online] 1 June 2001 (2001-06-01), "Hypothetical protein SA1754." retrieved from EBI accession no. UNIPROT:Q99SU9 Database accession no. Q99SU9_STAAN & DATABASE UniProt [Online] 1 June 2001 (2001-06-01), "Fibrinogen-binding protein." retrieved from EBI accession no. UNIPROT:Q99UU9 Database accession no. Q99UU9_STAAM & DATABASE UniProt [Online] 5 July 2004 (2004-07-05), "SA1004 protein." retrieved from EBI accession no. UNIPROT:Q7A636 Database accession no. Q7A636_STAAN & KURODA M ET AL: "Whole genome sequencing of meticillin-resistant Staphylococcus aureus" LANCET THE, LANCET LIMITED. LONDON, GB, vol. 357, no. 9264, 21 April 2001 (2001-04-21), pages 1225-1240, XP004246103 ISSN: 0140-6736
- IANDOLO J J ET AL: "Comparative analysis of the genomes of the temperate bacteriophages phi11, phi12 and phi13 of Staphylococcus aureus 8325" GENE: AN INTERNATIONAL JOURNAL ON GENES AND GENOMES, ELSEVIER SCIENCE PUBLISHERS, BARKING, GB, vol. 289, no. 1-2, 1 May 2002 (2002-05-01), pages 109-118, XP004358927 ISSN: 0378-1119 & DATABASE UniProt [Online] 1 June 2002 (2002-06-01), "Involved in expression of fibrinogen binding protein." retrieved from EBI accession no. UNIPROT:Q8SDJ7 Database accession no. Q8SDJ7_BPPHD & DATABASE EMBL [Online] 28 February 2002 (2002-02-28), "Staphylococcus aureus phage phi 13, complete genome." retrieved from EBI accession no. EM_PRO:AF424783 Database accession no. AF424783
- DATABASE EPO Proteins [Online] 20 February 2003 (2003-02-20), "Sequence 1328 from Patent WO02094868." XP002322489 retrieved from EBI accession no. EPOP:AX618365 Database accession no. AX618365 & DATABASE EPO Proteins [Online] 20 February 2003 (2003-02-20), "Sequence 1102 from Patent WO02094868." retrieved from EBI accession no. EPOP:AX618139 Database accession no. AX618139 & DATABASE EMBL [Online] 20 February 2003 (2003-02-20), "Sequence 1327 from Patent WO02094868." retrieved from EBI accession no. EM_PRO:AX618364 Database accession no. AX618364 & DATABASE EMBL [Online] 20 February 2003 (2003-02-20), "Sequence 1101 from Patent WO02094868." retrieved from EBI accession no. EM_PAT:AX618138 Database accession no. AX618138 & DATABASE Geneseq [Online] 20 November 2003 (2003-11-20), "Staphylococcus aureus protein #551." retrieved from EBI accession no. GSP:ABM71311 Database accession no. ABM7
- BABA T ET AL: "Genome and virulence determinants of high virulence community-acquired MRSA" LANCET, XX, XX, vol. 359, no. 9320, 25 May 2002 (2002-05-25), pages 1819-1827, XP004357882 ISSN: 0140-6736 & DATABASE UniProt [Online] 5 July 2004 (2004-07-05), "Hypothetical protein MW1884." retrieved from EBI accession no. UNIPROT:Q7A0F6 Database accession no. Q7A0F6 & DATABASE UniProt [Online] 5 July 2004 (2004-07-05), "MW1041 protein." retrieved from EBI accession no. UNIPROT:Q7A142 Database accession no. Q7A142_STAAW
- MATTHEW T.G. HOLDEN ET AL.: "Complete genomes of two clinical Staphylococcus aureus strains: Evidence for the rapid evolution of virulence and drug resistance" PROCEEDINGS OF THE NATIONAL ACADEMY OF SCIENCES USA, vol. 101, no. 26, 29 June 2004 (2004-06-29), pages 9786-9791, XP002322486 & DATABASE EMBL Entry BX571856 23 June 2004 (2004-06-23), HOLDEN, M.T.G. ET AL.: "Staphylococcus aureus subsp. aureus strain MRSA252, complete genome" Database accession no. BX571856
- S.H.M. ROOIJAKKERS ET AL.: "Lectin pathway inhibitor (LPI) of Staphylococcus aureus: a unique molecule that blocks phagocytosis" MOLECULAR IMMUNOLOGY, vol. 41, June 2004 (2004-06), page 301, XP002322487
- GÉZA AMBRUS ET AL.: "Natural substrates and inhibitors of Mannan-binding Lectin-associated Serine Protease-1 and -2: A study on recombinant catalytic fragments" THE JOURNAL OF IMMUNOLOGY, vol. 170, no. 3, 1 February 2003 (2003-02-01), pages 1374-1382,

## Description

The present invention relates to a (poly)peptide having Lectin Pathway Inhibitor (LPI) activity for use in the profylaxis and treatment of inflammatory reactions.

Complement is the complex network of over 20 serum proteins that are part of our innate immune system. Complement acts by itself (through lysis of microbes) or in conjunction with other components of the innate immune system (e.g. phagocytosis). Our innate immune system is mainly involved in protecting the body against foreign invaders (e.g. bacteria, viruses, fungi, and also cancer cells). The most important cells of the innate immune system are dendritic cells, monocytes/macrophages and neutrophils. Next to that, our innate immune system contains a large variety of soluble factors such as acute phase proteins, antimicrobial peptides, peptidases, parts of the clotting cascade and the complement system. Killing and removal of invaders is mostly done by monocytes and neutrophils, by direct recognition of the invaders or with the help of specific antibodies and/or the complement system (opsonization).

Cells of the innate system react in a relatively aggressive way. Since they are part of the body's first line of defense, their most important task is to kill and remove the invading agent as quickly as possible. This is accomplished through very aggressive substances (e.g. free radicals and enzymes) that are not only lethal to the invader, but also cause damage to host cells in the vicinity. Substances from these damaged cells and the locally activated cells from the innate system itself will further attract increasing numbers of neutrophils and monocytes, causing further local inflammation.

In most cases, such an aggressive and damaging inflammatory reaction, caused by over-activated neutrophils, is unnecessary. In some cases this inflammatory response is responsible for serious, sometimes lethal disorders and includes conditions like Adult Respiratory Distress Syndrome (ARDS), severe tissue damage following thrombotic events such as heart attacks and stroke, inflammatory bowel diseases and rheumatoid arthritis.

The inflammation will subside once all the invaders have been killed and removed, together with the various cells killed in the process. Healing of the wound, caused by the inflammatory response, can then begin. Although there is some overlap in function, the main task of neutrophils is to attack the invaders and the main task of monocytes is to remove the debris resulting from this attack. In addition, neutrophils have another peaceful task in assisting the wound healing process.

When bacteria have invaded the body, substances of microbial origin activate the complement system directly or via pre-existing antibodies. The first molecule involved in antibody-mediated complement activation is C1q followed by the activation of C1r and C1s. A parallel pathway does not need specific antibodies, because it directly recognizes microbial surface structures. H-ficolin, L-ficolin or Mannose Binding Lectin (MBL) recognize microbes and through activation of a specific MBL Associated Serine Proteases (MASP-2), the rest of the complement system is activated.

In both events activation proceeds through C4 and C2 and the central molecule of the complement system C3 is activated. This leads to more C3 deposition via the alternative pathway (factors B, D, H, I, P). C3 once converted into C3b, C3bi or even C3d is the most important opsonin, it mediates uptake of microbes by phagocytes, and importantly also activates these phagocytes in the process. Next to this phagocyte directed action, the complement system can proceed from C3 via C5, C6, C7, C8, and C9 to lysis of the tumor cell, virus infected cell, gram negative bacterium, or during unwanted inflammatory events, one of the healthy cells of our body.

Normally our cells are protected from unwanted complement attack by a variety of mechanisms (C1INH, C4bp, CR1, MCP, DAF, H, I, P, CD59) but in cases of disturbance or extremely high local activation, direct complement mediated damage can still occur. Furthermore, in the process of complement activation, the formation of opsonins and membrane attack is parallelled by the formation of very strong inflammatory small molecules (C5a, C3a). These substances directly activate phagocytes and other cells (via C5a and C3a receptors) in a very efficient way, resulting in damage to microbes or healthy cells. The interaction with different cell types also gives rise to the production of other chemokines (like interleukin-8, IL-8): substances that can activate and attract cells from the blood vessels (the migration process). Neutrophils interact with these substances, because they have receptors for these substances
on the outside of their cell membrane. An overview of the components of the complement system is given in Table 1.

**Table 1**

| PROTEINS INVOLVED IN THE COMPLEMENT CASCADE | |
|---|---|
| Binding to Ag:Ab complexes: | C1q |
| Direct recognition of microbial | |
| surface structures: | MBL, Ficolin-H, L-Ficolin, M-Ficolin |
| Activating Enzymes: | C1r, C1s, C2b, B, D, MASP1,2,3 |
| Membrane-binding opsonins: | C4b, C3b |
| Mediators of inflammation: | C5a, C3a, C4a |
| Membrane attack: | C5b, C6, C7, C8, C9 |
| Complement Receptors: | CR1, CR2, CR3, CR4, C1qR, M-Ficolin |
| Complement-regulatory proteins: | C1INH, C4bp, CR1, MCP, DAF, H, I, P, CD59 |

| | |
|---|---|
| *Adapted from Janeway & Travers Immunobiology, 1996; Current Biology Ltd/Garland Publishing Inc. | |

Activated neutrophils can easily migrate from blood vessels. This is because the chemokines and microbial products will have increased the permeability of the vessels and stimulated the endothelial cells of the vessel walls to express certain adhesion molecules. Neutrophils express selectins and integrins (e.g. CDllb/CD18) that bind to these adhesion molecules. This process is called priming. Once the neutrophil has adhered to the endothelial cells, it is able to migrate through the cells, under the guidance of chemokines, towards the site of infection, where the concentration of these substances is at it's highest.

These substances also activate neutrophils to produce a range of other molecules, some of which attract more neutrophils (and subsequently monocytes), but, mostly, they are responsible for destroying the invading bacteria. Some of these substances (e.g. free radicals, enzymes that break down proteins (proteases) and cell membranes (lipases)) are so reactive and non-specific that cells from the surrounding tissue (and the neutrophils themselves) are destroyed, causing tissue damage. This damage is exacerbated by the presence of blood-derived fluid, which has transgressed the leaky vessel wall and is responsible for the swelling that always accompanies inflammation (called edema). The pressure build up caused by this excess fluid results in further cell damage and death.

The onset of an inflammatory reaction does not have to be of microbial origin per se. Tissue damage in general, by oxygen deprivation, pH changes, salt disbalance or physical damage can start inflammatory reactions. In many cases the key event is the activation of the complement system. In autoimmune diseases, the presence of auto-antibodies gives rise to the activation of the classical pathway of complement. In almost all other events the activation of complement is via the lectin pathway (cf. Jordan et al., Circulation. 2001,104(12):1413-8; Collard et al., Am J Pathol. 2001, 159:1045-54; Roos et al., J Immunol. 2001, 167:2861-8; Collard et al., Am J Pathol. 2000, 156:1549-56; Collard et al., Mol Immunol. 1999,36:941-8). Therefore the lectin pathway of complement activation is of crucial importance as the first step in many inflammatory conditions and diseases.

Later in the inflammatory process, monocytes migrate to the scene and become activated. Besides their role in removing bacteria and cell debris, they also produce substances such as tumor necrosis factor (TNF) and IL-8, which in turn attract more activated neutrophils, causing further local damage. TNF also has a direct stimulatory effect on neutrophils. Once all the invaders have been removed, the inflammatory response will subside and the area will be cleared of the remaining "casualties". Then the process of wound healing starts. Although it is known that neutrophils play a pivotal role in wound healing, it is not clear which neutrophil-derived substances are involved and how the neutrophils are active in healing without being aggressive to the surrounding tissue. In general, damaged tissue will be replaced by scar tissue formed mainly of fibroblasts and collagen.

When inflammation occurs in areas of the body with an important function, like tissues formed from heart muscle cells, brain cells or lung alveolar cells, normal function will be compromised by the resulting scar formation, causing serious conditions like heart failure, paralysis and emphysema. To minimize the debilitating consequences of these conditions, it is important to "dampen" the inflammatory reaction as quickly as possible.

Intervention to control the acute early phase inflammatory response presents an opportunity to improve the prognosis for a wide range of patients whose symptoms can be traced back to such an event. Such an approach has been advocated for many acute and chronic inflammation based diseases and shown to have potential based on findings from relevant disease models. Classical anti-inflammatory drugs such as steroids and Non Steroid Anti-Inflammatory Drugs (NSAIDS) do not have the ideal profile of action, either in terms of efficacy or safety. Steroids affect the "wrong" cell type (monocytes) and their dampening effects are easily bypassed. NSAIDS generally show a relatively mild effect partly because they intervene at a late stage in the inflammatory process. Both classes of drugs produce a range of undesirable side effects resulting from other aspects of their pharmacological activity.

Several drugs under early development only interfere with late mediators in the route to neutrophil activation (e.g. C5 convertase inhibitors, antibodies against C5a, C5a-receptor blocking drugs, antibodies against integrins (like CD11b/CD18) and L-selectin on neutrophils and antibodies against adhesion molecules (like ICAM-1 and E-selectin) on endothelial cells).

Antibodies against TNF and IL-8 have effects in chronic inflammation, but only marginal effects in acute inflammation, because of the minimal role monocytes (which are mainly responsible for these substances' production) play in the acute phase and because they react even later in the inflammation cascade. In many cases it would be extremely desirable to stop the inflammatory cascade in an early-as-possible-phase. This is also true because this cascade is not linear but branches off at different stages causing redundancy in the later steps.

Sometimes, the cause of the acute inflammation cannot be removed and the inflammation becomes chronic. With the exception of tuberculosis, chronic hepatitis and certain other conditions, this is seldom the case with infections. However, chronic inflammation can also be caused by stimuli other than bacteria, such as auto-immune reactions. Research has shown that in chronic inflammation the role of monocytes is much more prominent, and that neutrophil migration and activation, monocyte migration and activation, tissue damage, removal of dead cells and wound healing are all going on at the same time.

This complex cascade of interactions between cells and many different cytokines and chemokines has been the subject of intensive research for many years. It was believed that monocytes and their products were the most important elements that needed to be inhibited to dampen chronic inflammation. This explains why steroids, which specifically interact with monocytes, are generally more effective in chronic as opposed to acute inflammation. Long-term treatment with steroids however, is not a desirable option, because severe and unacceptable side effects can occur at the doses required to produce a clinical effect.

Newer treatments using antibodies against TNF or IL-8 have shown good results, and were initially seen as proof of the major role monocytes were thought to play in chronic inflammation. Recent research casts doubts on an exclusive role for monocytes in inflammation and points to a critical role for neutrophils, which are now seen to represent better targets for therapeutic intervention. Also in chronic inflammation it could still be desirable to dampen the early onset of activation as opposed to totally block one of the later steps. This early step-treatment (e.g. lectin pathway inhibition) could lead in due course to modification of disease progression, or even a complete cure, and not just symptomatic relief.

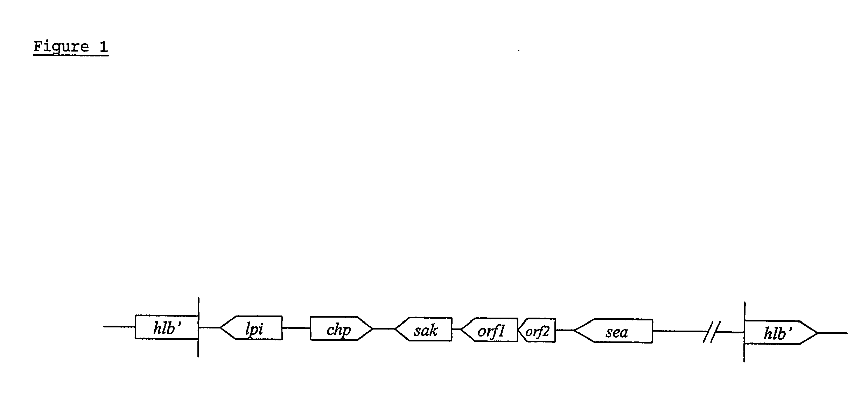

In the research that led to the present invention the gene, the (poly)peptide and its function for a new agent with inflammation-inhibiting properties was found in the bacterium *Staphylococcus aureus (S. aureus).* Recently, the inventors described a neutrophil modulating agent, CHIPS (Chemotaxis Inhibitory protein of Staphylocci as described in PCT/NL99/00442, which was found to be located on a bacteriophage. On this phage, a so-called Pathogenicity Island was found containing four genes, the genes for CHIPS (chp), Staphylokinase (sak) and enterotoxin A (sea) and a fourth unknown open reading frame (figure 1).

The inventors found that the three known genes are virulence factors that have one thing in common. They all interfere with the innate immune system. CHIPS, as they found, inhibits chemotaxis towards C5a and fMLP. Staphylokinase interferes via human plasmin, as the inventors have shown recently, with IgG mediated opsonization and also with complement mediated opsonization. Furthermore, others recently described that SAK causes destruction of defensins, important antimicrobial peptides. Enterotoxin A is described as an superantigen to interfere with adaptive immunity but others also found that it blocks the response to certain chemokines by modulating chemokine receptors.

From this the inventors concluded that the fourth open reading frame within SaPI-5, with unknown function was very likely to encode a protein that would also interfere with innate immunity. The hypothesis was that this open reading frame would inhibit some innate immune mechanism, important to fight staphylococcal infections in one way or another.

To prove this hypothesis the inventors cloned and expressed the protein encoded in this open reading frame in *E. coli* and purified the protein to homogeneity. This pure protein was evaluated in a number of their *in vitro* innate immunity assays, in particular: chemotaxis, chemokinesis, cytokine induction (TNF, IL-1, IL-6, IL-10), chemokine induction in whole blood or in isolated monocytes or mononuclear cells, Ca-flux assays with neutrophils or mononuclear cells and flow cytometry, adherence assays (fluorometer), transmigration of neutrophils through endothelium (fluorometric), actin polymerisation (flow cytometrie), phagocytosis (uptake) of standard opsonized erythrocytes (flow cytometry), phagocytosis (opsonization) of standard neutrophils with different opsonins and different bacteria, quantitative bacterial killing assays, membrane depolarization assays (FLEX station), metabolic burst measurements in a luminometer (production O₂-radicals), priming assays for fMLP, PAF etc (luminometer), degranulation assays for MPO and elastase (FLEX station), receptor expression assays (neutrophil/monocyte phenotyping for innate immune receptors, flow cytometry)etc. Hereafter it was concluded that LPI is indeed a modulator of innate immunity. It specifically inhibits the lectin pathway of complement activation. Therefore the protein was named LPI (Lectin Pathway Inhibitor).

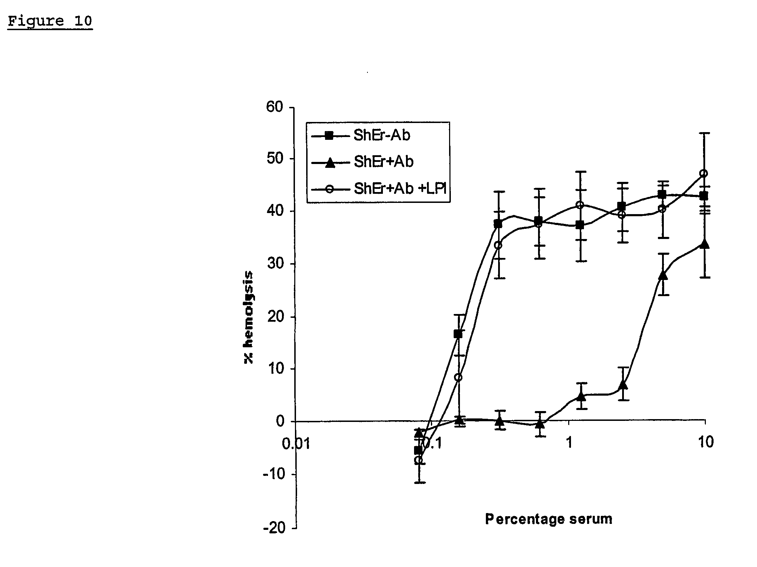

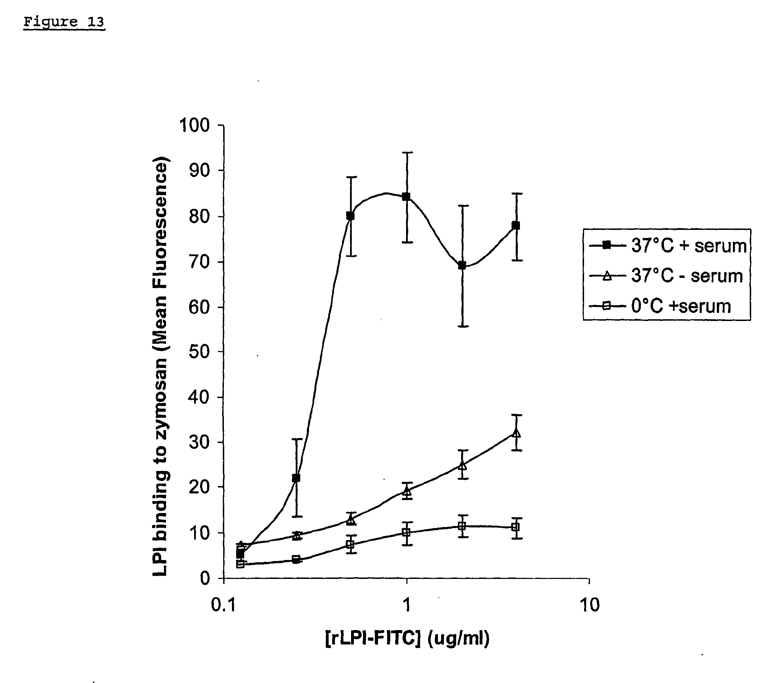

Thus LPI was designed by staphylococcal evolution, specifically to inhibit the lectin pathway of complement activation. This is the pathway, which is the greatest threat to staphylococci in the early phases once they have invaded the human body. Both MBL and ficolins can directly recognize the staphylococcal cell wall and initiate the complement cascade, leading to the attraction of neutrophils (C5a, C3a) and the opsonization of staphylococci. For this reason the inventors also demonstrated the activity of LPI in phagocytosis of staphylococci in Example 3. After evaluating LPI in all separate pathways of the complement cascade the inventors concluded that LPI is a specific lectin pathway inhibitor **(****figures 10****,** **11** **and** **12****)** After evaluating LPI in all separate steps in the lectin pathway they concluded that LPI inhibits lectin pathway mediated complement activation by inhibiting the C2 cleavage activity of MASP-2 but not the C4 cleavage activity of MASP-2 **(****figures 13****,** **14****,** **15****,** **16****,** **17****,** 18, **19****,** **20****).**

Therefore LPI activity is defined as follows: LPI prevents activation of the lectin pathway of complement activation by specifically preventing the MASP-2 dependent cleavage of C2 into C2a and C2b.

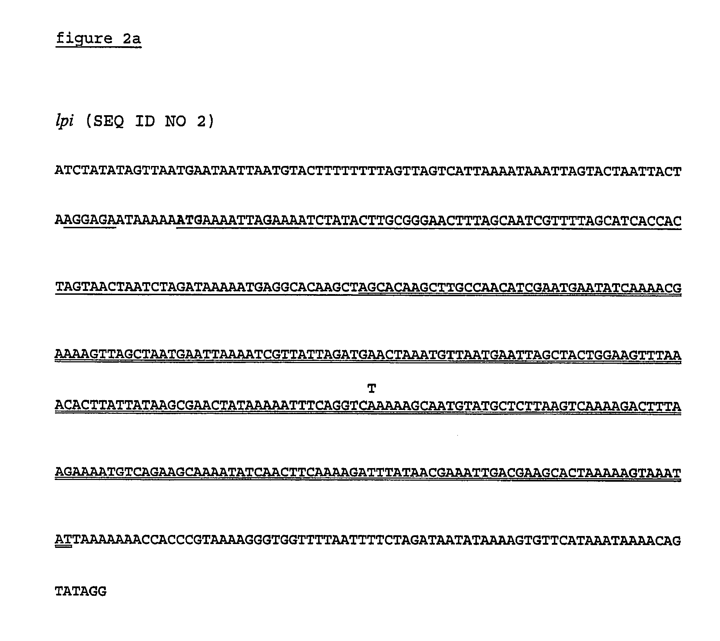

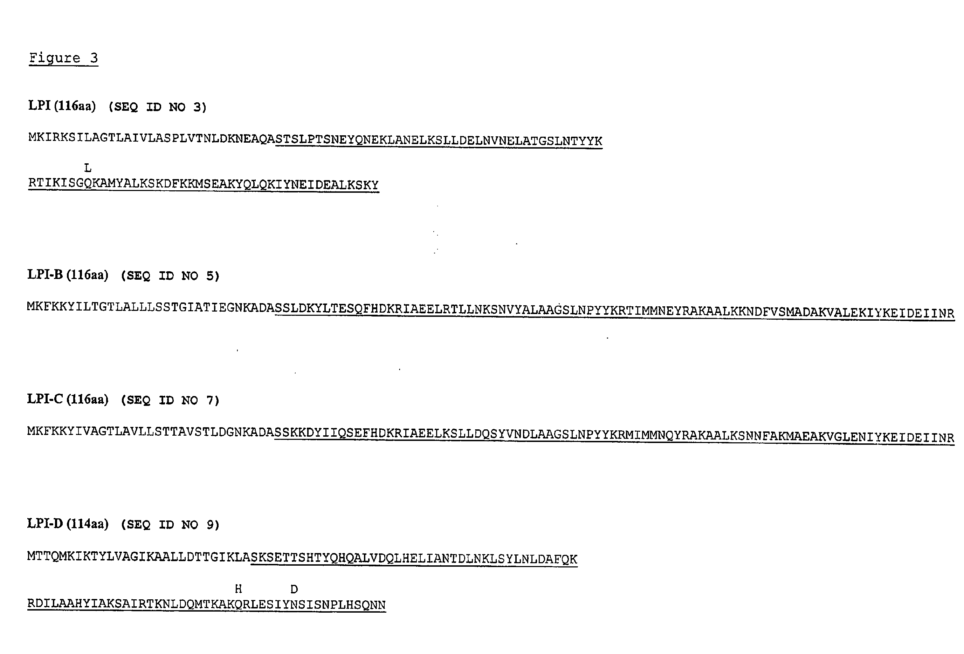

Next to the gene for LPI **(****figure 2a****, SEQ ID NO:2)** three other homologues genes were identified, also from *S. aureus* designated lpi-B **(SEQ ID NO:4),** lpi-C **(SEQ ID NO:6)** and lpi-D **(SEQ ID NO:8).** These genes were cloned and expressed and tested for lpi activity. The proteins encoded by these genes are LPI-B **(SEQ ID NO:5),** nd LPI-C **(SEQ ID NO:7)** and LPI-D **(SEQ ID NO:9) (****figure 3****).** It was concluded that LPI-B and LPI-C have LPI-like activity. LPI-D has lower homology and no LPI activity.

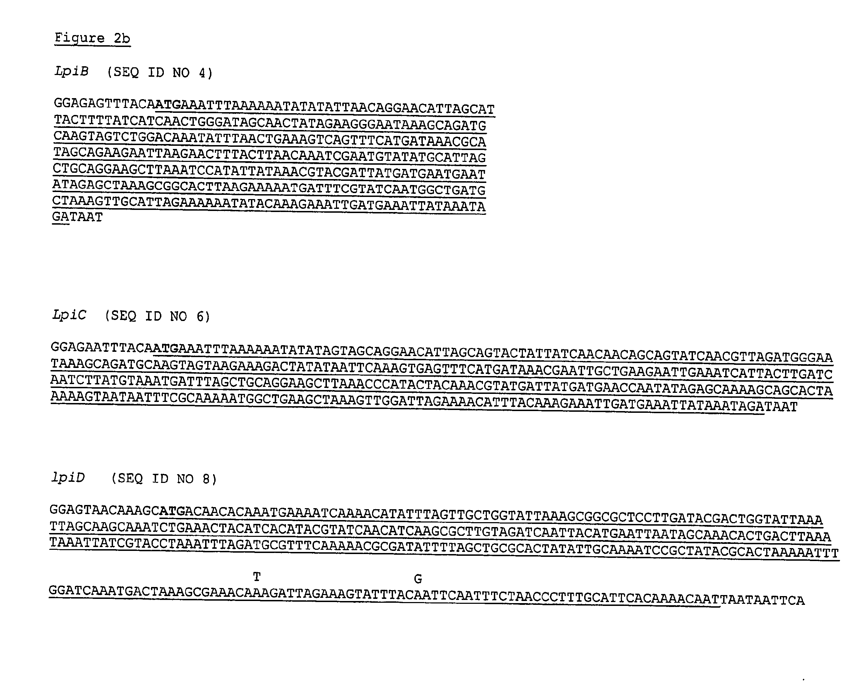

The present invention therefore provides a peptide or polypeptide encoded by a nucleotide sequence corresponding to a sequence being selected from the group consisting of:
a) a nucleotide sequence comprising a part of one of the sequences as depicted in **Figure 2a** and **2b** and identified as **SEQ ID NO:2; SEQ ID NO:4; SEQ ID NO:6);**
b) nucleotide sequences encoding a peptide or polypeptide having LPI activity and having the amino acid sequence depicted in **Figure 3** and identified as **SEQ ID NO:3,**
   **SEQ ID NO:5** or **SEQ ID NO:7;**
c) nucleotide sequences encoding a peptide or polypeptide having LPI activity and having a portion of the amino acid sequence depicted in **Figure 3** identified as **SEQ ID NO:3, SEQ ID NO:5** or **SEQ ID NO:7;**
d) nucleotide sequences being at least 40% identical to any one of the nucleotide sequences a), b) or c);
e) nucleotide sequences hybridizing at stringent conditions with any one of the nucleotide sequences a), b), c) or d), and
f) nucleotide sequences complementary to any of the nucleotide sequences a), b), c), d) or e),
wherein said peptide or polypeptide has LPI (Lectin Pathway Inhibitor) activity which prevents activation of the lectin pathway of complement activation by specifically preventing the MASP-2 dependent cleavage of C2 into C2a and C2b,
and said peptide or polypeptide is for use in profylaxis or treatment of acute and chronic inflammation reactions.

WO94/06830 already discloses a *Staphylococcus aureus* binding protein showing 100% identity with SEQ ID NO:5. The protein is used for immunizing mice against *S. aureus* infection and to block infection in wounds. The MASP-2 dependent cleavage of C2 into C2a and C2b and its use for the prevention or treatment of inflammation is not disclosed in this document.

The complete genome of *Staphylococcus aureus* was sequenced and can be found in the regular international databases like GenBank, RefSeq, and PDB. The above sequences identified by SEQ ID NOS 2, 4 and 6 can be found in the database as part of a larger sequence. The nucleic acid in isolated form as related to the described function is however novel. The accession numbers for the hypothetical proteins that are now identified as LPI are gi|14247715|dbj|BAB58104.1| and gi|13701735|dbj|BAB43028.1|. LPI encodes a putative protein of 116 amino acids which shares 35-45% homology with other *Staphylococcus aureus* proteins of the same size, LPI-B (gi|13700958|dbj| BAB42254.1|; gi|14246929|dbj|BAB57321.1|), and LPI-C (gnl|sanger_159288|Staphylococcus (from the Sanger database(*S. aureus* 252, (MRSA 16)).

These sequences are depicted in **figure 2b** (lpi-B, **SEQ ID NO:4,** lpi-C, **SEQ ID NO:6).**

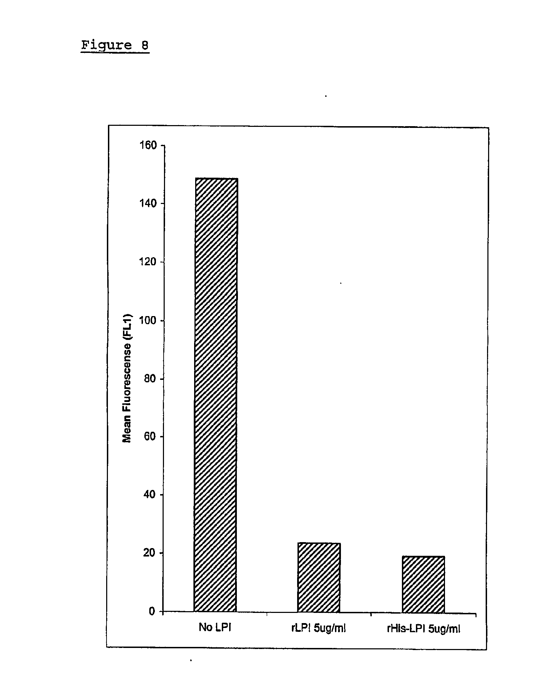

It was shown that the proteins encoded by the homologous genes lpi-B and lpi-C have at least LPI activity **(figure 8b).**

Regarding the term "identical" under d) above it should be noted that identicity and homology are used interchangeably. It should furthermore be noted that for gapped alignments, statistical parameters can be estimated using the Smith-Waterman algorithm that produces optimal alignment scores. Homologues of the LPI nucleic acid sequence or protein sequence are defined by a Gap Open Penalty of at least 12 and a Gap Expression Penalty of at least 1.

The sequence as given in **Figure 2a** **(SEQ ID NO:2) is** one embodiment of the DNA sequence encoding the (poly)peptide of the invention. It comprises a promoter region from nucleotides 1 to 86, a leader peptide sequence from nucleotides 87 to 179, the coding region for the (poly)peptide having LPI activity from nucleotide 180 to 434, as well as a 3' untranslated region from nucleotides 435 to 510.

The presented gene for LPI of **Figure 2a** or any nucleic acid derived therefrom may for example be operably linked to the trc expression system (Brosius et al., Gene 27: 161-172 (1984)). Many other suitable expression control sequences and methods of expressing recombinant proteins are known (F.M. Ausubel et al., Current Protocols in Molecular Biology, John Wiley and Sons, Inc., New York, N.Y.).

The nucleotide sequence as given in **Figure 2a** also contains a leader peptide sequence. The coding region of the mature protein corresponds to nucleotides 180 to 434 of **Figure 2a****.** Other leader sequences can be used. Or the leader sequence may be omitted entirely, depending on the host cell in which the sequence is to be expressed.

The amino acid sequence in **Figure 3** (LPI) **(SEQ ID NO:3)** is deduced from the DNA sequence in **Figure 2a****.** In a further embodiment of the invention the nucleic acid molecule encoding the (poly)peptide thus may have a nucleotide sequence that corresponds to all degenerate variants of the LPI gene, the lpi-B gene or the lpi-C gene.

The invention furthermore relates to (poly)peptides encoded by nucleic acid molecules that do not have the complete sequences LPI **(SEQ ID NO:3),** LPI-B **(SEQ ID NO:5)** or LPI-C **(SEQ ID NO:7)** from **figure 3** but one or more functional portions thereof that in themselves or together constitute a biologically active (poly)peptide having LPI activity. "A portion" as used herein does not exclude the possibility that a (poly)peptide comprises more than one portion and should thus be interpreted as "at least one". Such portions may vary in size from the complete amino acid sequence minus one amino acid to peptides of at least 2, preferably at least 5 amino acids. In case the active part of the protein lies in two or more portions of the complete amino acid sequence, the invention also relates to nucleic acid sequences encoding these separate portions in a manner that leads to a peptide configuration that retains the biological activity. In practice this can for example mean that spacer sequences are to be incorporated in between biologically active portions to lead to a biologically active conformation.

Thus, when reference is made to "at least part of the sequence" this means not only the three parts described above (i.e. for LPI: nucleotides 1-434, 87-434 and 180-434) but also other fragments of the gene or combinations thereof provided that they still encode a (poly)peptide having LPI activity.

In a further embodiment thereof, the invention thus relates to a (poly)peptide encoded by an isolated nucleic acid molecule which consists of the coding region of one or more portions of the amino acid sequence LPI **(SEQ ID NO:3),** LPI-B **(SEQ ID NO:5)** or LPI-C **(SEQ ID NO:7)** from **figure 3****,** wherein one portion of the amino acid sequence constitutes alone or with other portions of the amino acid sequence the region(s) of the (poly)peptide having LPI activity that lead to biological activity.

The present invention is not limited to (poly)peptides encoded by nucleic acid molecules having the exact same sequence as the sequence lpi **(SEQ ID NO:2),** lpi-B **(SEQ ID NO:4)** or lpi-C **(SEQ ID NO:6)** depicted in Figure 2a and 2b or the above described variants thereof. Therefore, according to the invention the (poly)peptides can be encoded by additional nucleic acid molecules having a nucleotide sequence which is at least 40%, preferably at least 50%, more preferably at least 60%, even more preferably at least 70%, most preferably at least 80%, the most preferable at least 90% identical or homologous to any one of the nucleotide sequences as defined under a), b) or c) above. Homology is to be determined over the entire length of the homologous sequence.

It was found that LPI is less than 40% homologous to proteins and peptides known to date. Proteins and peptides that show at least 40% amino acid homology to the LPI protein and have LPI activity are thus also part of this invention.

The invention further relates to (poly)peptides encoded by nucleic acid molecules having a nucleotide sequence hybridizing under stringent conditions with a nucleic acid molecule corresponding with the nucleotide sequence lpi **(SEQ ID NO:2),** lpi-B **(SEQ ID NO:4)** or lpi-C **(SEQ ID NO:6)** given in **Figure 2a** or **2b** or degenerate sequences thereof, which encode an amino acid sequence LPI **(SEQ ID NO:3),** LPI-B **(SEQ ID NO:5)** or LPI-C **(SEQ ID NO:7)** as given in **Figure 3****.** Stringent conditions are constituted by overnight hybridization at 42°C in 5xSSC (SSC = 150 mM NaCl, 15 mM trisodium citrate) and washing at 65°C at 0.1xSSC. In addition to 5xSSC the hybridization solution may comprise 50% formamide, 50mM sodium phosphate (pH 7.6), 5x Denhardt's solution, 10% dextran sulphate and 20 mg/ml denatured sheared salmon sperm DNA.

The invention is also not limited to (poly)peptides encoded by the gene which encodes the (poly)peptide having LPI activity, but also relates to nucleic acid molecules that encode fragments, derivatives and analogues thereof. "Fragments" are intended to encompass all parts of the (poly)peptide that retain its biological activity. "Fragments" can consist of one sequence of consecutive amino acids or of more than one of such sequences. "Derivatives" are the complete (poly)peptide having LPI activity or fragments thereof that are modified in some way. Examples of modifications will follow herein below. One example is enabled in Example 3, the His-tagged LPI molecule has also LPI activity. "Analogues" are similar (poly)peptides having LPI activity isolated from other organisms, in particular other pathogenic organisms. All of the above categories have one thing in common, namely that they have " LPI activity". LPI activity can be measured by any assay that shows inhibition of complement activation. Examples of such assays include deposition of C2, C4 or C3 fragments on a bacterium or erythrocyte, CH50 measurements using lysis of erythrocytes as a readout and others. Therefore, for the present application, the term "(poly)peptides having LPI activity" is intended to include the original LPI, LPI-B and LPI-C proteins and their homologues in isolated or recombinant form, and other (poly)peptides, fragments, derivatives and analogues that exhibit LPI activity.

Also disclosed herein are probes and primers derived from the nucleic acid molecule of the invention. Such primers are oligonucleotides or polynucleotides of at least about 10 consecutive nucleotides (nt), and more preferably at least about 25 nt, still more preferably at least about 30 nt, and even more preferably about 30-70 nt of the nucleic acid molecule of the invention. Probes are longer and may for instance be a portion of the nucleic acid molecule of the invention of 50-300 consecutive nt, or even as long as the entire nucleic acid molecule.

Such oligonucleotides or polynucleotides are useful as diagnostic probes or as probes in conventional DNA hybridization techniques or as primers for amplification of a target sequence by polymerase chain reaction (PCR) as described for instance in Ausubel et al. (*supra*) or other amplification techniques, such as NASBA.

Furthermore, a recombinant vector is disclosed herein comprising at least one isolated nucleic acid molecule of the invention. The vector to be used can be selected by the skilled person based on his common general knowledge and will be dependent on the host that is used.

In addition to vectors, a bacteriophage is disclosed comprising at least one isolated nucleic acid that encodes the (poly)peptide of the invention. In most LPI-positive Staphylococci, the gene encoding LPI is located on a prophage and can be turned into an active phage, for example by treatment with mitomycin-C according to standard and published phage isolating procedures. A bacteriophage is thus a useful vehicle to introduce the LPI gene into a host.

In addition a method is disclosed for making a recombinant vector, comprising inserting at least one isolated nucleic acid molecule of the invention into a vector. By incorporating more than one copy in the vector, or introducing more than one vector into a host, the level of expression can be influenced. When a host cell is used that comprises an endogenous gene for a corresponding (poly)peptide having LPI activity, the expression level thereof can be increased by introducing more copies of the nucleic acid molecule (i.e. the gene) into the host cell or changing the promoter or regulator regions.

Also disclosed are recombinant host cells comprising at least one isolated nucleic acid molecule or vector . A number of types of organisms or cells from prokaryotes, protists, fungi, animals or plants may act as suitable hosts for the expression of recombinant (poly)peptides having LPI activity. Host cells include the widely used bacterial strain *Escherichia coli (E. coli)* including, but not limited to, the trc expression system (Brosius et al., *supra*) that allows high-level, regulated expression from the trc promotor. Potentially suitable other bacterial strains include Gram-positive bacterial strains, such as *Bacillus subtilis, Staphylococcus aureus,* or any bacterial strain capable of expressing heterologous proteins. A preferred production process in *E.coli* is given in Example 2.

The (poly)peptide having LPI activity may also be produced as a recombinant protein using a suitable expression system employing lower eukaryotes such as yeast or insect cells. Suitable yeast strains include *Saccharomyces cerevisiae, Pichia pastoris, Candida* or any yeast strain capable of expressing heterologous proteins. Insect cells used for recombinant protein expression include the *Drosophila* system and the *Baculovirus* system. Alternatively, it may be possible to produce the (poly)peptide having LPI activity in an mammalian expression system that includes several suitable host cells, including monkey COS, hamster CHO, BHK or RBL-2H3, human 293, 3T3, HeLa, U937, HL-60, or Jurkat cells, mouse L cells and other transformed cells for *in vitro* culture. For expression of (poly)peptides having LPI activity in eukaryotic systems, it may be necessary to modify the protein produced therein in order to obtain a functional protein. Such modifications, like attachments or substitutions may be accomplished using known chemical or enzymatic methods. In addition, the sequence of the nucleic acid molecule may be adapted to the codon usage of the host cell.

The (poly)peptide having LPI activity of the invention may also be expressed as a product of transgenic animals, e.g. as a component of the milk of transgenic cows, goats, pigs, sheep, rabbits or mice which are characterized by somatic or germ cells containing a nucleotide sequence encoding the (poly)peptide having LPI activity.

Alternatively the (poly)peptide having LPI activity may be expressed in a form that will facilitate purification. For example, it may be tagged with a polyhistidine (6xHis) epitope and subsequently purified by using a resin to which nickel ions are bound by means of a chelating agent. The (poly)peptide having LPI activity containing the tag is eluted from the resin by lowering pH or by competing with imidazole or histidine. Such epitope is commercially available from Invitrogen (Breda, The Netherlands). Introduction of a protease cleavage site, like that for enterokinase, enables removal of the fusion tag to generate mature native recombinant (poly)peptide having LPI activity. Materials and methods for such an expression system are commercially available from Invitrogen, using the pTrcHis Xpress^{™} vectors in combination with ProBound^{™} resin for efficient isolation of His-tagged protein and EnterokinaseMax^{™} as highly catalytic active protease and EK-Away^{™} enterokinase affinity resin to remove the contaminating presence of the protease. Other tags known to those skilled in the art that can be used to facilitate purification include, but are not limited to, glutathion S transferase (GST fusion), myc and HA.

The (poly)peptide having LPI activity may also be produced by known chemical synthesis. Methods for constructing polypeptides or proteins by synthetic means are known to those skilled in the art. The synthetic protein, by virtue of sharing primary, secondary and tertiary structural and/or conformational characteristics with the corresponding (poly)peptide having LPI activity will posses an activity in common therewith, meaning LPI properties. Thus, such synthetically produced proteins can be employed as biologically active or immunological substitute for natural purified (poly)peptide having LPI activity.

The (poly)peptides having LPI activity provided herein also include (poly)peptides characterized by amino acid sequences into which modifications are naturally provided or deliberately engineered. Modifications in the (poly)peptide or DNA sequences can be made by those skilled in the art using known conventional techniques. Modifications of interest in the LPI active (poly)peptide sequences may include replacement, insertion or deletion of selected amino acid residues in the coding sequence.

The information contained in the LPI protein, its gene and other (poly)peptides having LPI activity and their encoding nucleic acid molecules derived therefrom can be used to screen for fragments thereof or other agents which are capable of inhibiting or blocking binding of a (poly)peptide having LPI activity in complement activation assays, and thus may act as inhibitors of LPI binding to its putative target. Appropriate screening assays may for example use the fluorescent labelled purified LPI protein that binds to bacteria in the presence of an intact complement system and analysed by flow cytometry or fluorometry. A suitable binding assay may alternatively employ purified LPI-target or target-domain on a carrier with a form of LPI protein as binder. Alternatively, an assay can be employed that screens for the ability to bind or compete with LPI for binding to a specific anti-LPI antibody (monoclonal, polyclonal, or single chain antibody) by various immunoassays known in the art, including but not limited to competitive and non-competitive ELISA techniques or Biosensor technology employing a sensor chip coated with either ligand (LPI), antibody or putative LPI target (Surface Plasma Resonance (SPR) technique like the BiaCore). Any (poly)peptide having LPI activity other than LPI may also be used in the screening assays described. All these methods can be adapted for High Throughput Screening (HTS).

The functional activity of LPI, the (poly)peptides, their fragments, derivatives and analogues can be assayed by various methods. Al methods that measure complement activation at one of its steps can be used as a readout because LPI interferes with the first steps in this process. Thus, C4, C2, or C3 fragments-deposition (by flow cytometry or ELISA or immunoblotting), CH50 measurements using erythrocyte lysis, measurement of MAC complex or soluble split products of the complement cascade (ELISA or functional assays) are all suitable candidates for measuring LPI activity. Examples 3, 4, 5, 7 and 8 describe such methods..

Isolated (poly)peptides having LPI activity may be useful in treating, preventing or ameliorating inflammatory conditions that are involved in many diseases and disorders, such as those listed in **Table 2.**

According to a further aspect thereof, the invention thus relates to (poly)peptides having LPI activity for use in diagnosis, prophylaxis or therapy, in particular for use in the treatment of acute and chronic inflammation reactions, such as those listed in **Table 2.**

**Table 2**

| Diseases caused by inflammatory reactions, involving complement activation and/or neutrophil and or monocyte involvement. | |
|---|---|
| acute reactive arthritis | bullos pemphigoid |
| acute transplant rejection | burn injuries |
| adult respiratory distress | burns |
| syndrome (ARDS) | cardiopulmonary bypass |
| alcoholic hepatitis | cardiovascular diseases |
| allergic rhinitis | chronic bronchitis |
| allotransplantation | chronic lymph leukemia |
| Alzheimer's disease | chronic obstructive pulmonary |
| arteriosclerosis | disease (COPD) |
| arthus reaction | contact dermatitis |
| asthma | Crohn's disease |
| atherosclerosis | cutaneous T-cell lymphoma |
| atopic dermatitis | cystic fibrosis |
| bacterial meningitis | dermatoses |
| bacterial pneumonia | diseases of the central nervous |
| brain tumour | system |
| bronchogenic carcinoma | endometriosis |
| experimental allergic | pancreatitis |
| encephalomyelitis (EAE) | peritonitis |
| experimental allergic neuritis | pleural emphesema |
| (EAN) | post- cardiopulmonary bypass |
| Forssman shock | (CBP) inflammation |
| frost bite | psoriasis |
| gastric carcinoma | repetitive strain injury (RSI) |
| gastrointestinal diseases | respiratory diseases |
| genitourinary diseases | rheumatoid arthritis |
| glomerulonephritis | sepsis |
| gout | septic shock |
| haemolytic anemia | sinusitis |
| Heliobacter pylori gastritis | skin diseases |
| hemodialysis | stroke |
| hereditary angioedema | systemic lupus erythematosus |
| hypersensitivity pneumonia | (SLE) |
| idiopathic pulmonary fibrosis | transplantation |
| immune complex (IC)-induced | (traumatic) brain injury |
| vasculitis | Trichomonas vaginalis infection |
| ischaemic shock | ulcerative colitis |
| ischaemia-reperfusion episodes | urinary tract infection |
| ischemia-reperfusion injuries | vascular leak syndrome |
| joint diseases | vasculitis |
| (large) vessel surgery | viral hepatitis |
| metal fume fever | viral meningitis |
| multiple sclerosis | viral respiratory tract |
| multiple system organ failure | infection |
| myasthenia gravis | xenotransplantation |
| Mycobacterium tuberculosis | |
| infection | |
| myocardial infarction | |

| | |
|---|---|
| * Support for the therapeutical usefulness of the (poly)peptides of the invention for treatment of these diseases can be found in the following references: For ARDS: Demling RH (1995). The modern version of adult respiratory distress syndrome. Ann. Rev. Med. 46:193-202; and Fujishima S, Aikawa N 1995 Neutrophil mediated tissue injury and its modulation. Intensive Care Med 21:277-285; For severe infections (meningitis): Tunkel AR and Scheld WM (1993). Pathogenesis and pathophysiology of bacterial meningitis. Clin. Microbiol. Rev. 6:118. For injury after ischaemia/reperfusion: Helier T, et al. (1999). Selection of a C5a receptor antagonist from phage libraries attenuating the inflammatory response in immune complex disease and ischemia/reperfusion injury. J. Immunol. 163:985-994. For rheumatoid arthritis: Edwards SW and Hallett MB (1997). Seeing the wood for the trees: the forgotten role of neutrophils in rheumatoid arthritis. Immunology Today 18: 320-324; and Pillinger MH, Abramson SB (1995). The neutrophil in rheumatoid arthritis. Rheum. Dis. Clin. North Am. 1995 21:691-714. For myocardial infarction: Byrne JG, Smith WJ, Murphy MP, Couper GS, Appleyard RF, Cohn LH (1992). Complete prevention of myocardial stunning, contracture, low reflow, and edema after heart transplantation by blocking neutrophil adhesion molecules during reperfusion. J. Thorac. Cardiovasc. Surg. 104:1589-96. For COPD: Cox G (1998). The role of neutrophils in inflammation. Can. Respir. J. 5 Suppl A:37A-40A; and Hiemstra PS, van Wetering S, Stolk J (1998). Neutrophil serine proteinases and defensins in chronic obstructive pulmonary disease: effects on pulmonary epithelium. Eur. Respir. J. 12:1200-1208. For stroke: Barone FC, Feuerstein GZ (1999). Inflammatory mediators and stroke: new opportunities for novel therapeutics. J. Cereb. Blood Flow Metab. 19:819-834; and Jean WC, Spellman SR, Nussbaum ES, Low WC (1998). Reperfusion injury after focal cerebral ischemia: the role of inflammation and the therapeutic horizon. Neurosurgery 43:1382-1396. For meningitis: Tuomanen EI (1996). Molecular and cellular mechanisms of pneumococcal meningitis. Ann. N. Y. Acad. Sci. 797:42 52. For all directly complement related diseases: Adapted from: A. Sahu and J.D. Lambris Immunopharmacology 49 (2000) 133-148. | |

The invention furthermore relates to the use of the (poly)peptides having LPI activity for the manufacture of a preparation for diagnosis, prophylaxis or therapy, in particular for the treatment of acute and chronic inflammation reactions, more in particular for the treatment of the indications referred to above.

Also part of the present invention are therapeutic compositions comprising a suitable excipient and one or more of the (poly)peptide having LPI activity of the invention. Such composition can be used for the treatments as specified above.

The nucleic acid molecule of the invention, optionally incorporated in a larger construct, may be used for various purposes, such as raising antibodies thereto, modulating the LPI activity or in a therapeutic preparation.

The invention further relates to the amino acid sequence encoded by the nucleic acid molecules that can be identified by so-called "computer cloning". More specifically, this technique comprises using:
(1) the nucleic acid sequence lpi **(SEQ ID NO:2),** lpi-B **(SEQ ID NO:4)** or lpi-C **(SEQ ID NO:6)** as depicted in figure 2, or fragments, derivatives and analogues thereof, or
(2) the amino acid sequence LPI **(SEQ ID NO:3),** LPI-B **(SEQ ID NO:5)** or LPI-C **(SEQ ID NO:7)** as depicted in **Figure 3****,** or fragments, derivatives and analogues thereof, as a query for screening nucleic acid sequences or nucleic acid sequence databases, or protein sequences or protein sequence databases, using search algorithms that can identify regions with homology. Such algorithms are known to the person skilled in the art and include, but are not limited to, BLAST searches (Altschul et al., J. Mol. Biol. 215, 403-410 (1990)). The sequence databases that may be searched include, but are not limited to, the Genbank^{™} database and the Swissprot^{™} database. When using a BLAST search or modifications thereof, generally subjects that display homology can be identified. Identification is based on the value of the Score or the Smallest Sum Probability P(N). Homologues of the LPI nucleic acid sequence or (poly)peptide sequence are defined by a Score that is at least 200, preferably at least 400, more preferably at least 800, most preferably at least 1600. Alternatively, the P(N) value can be used for identification of homologous sequences. Homologues of the LPI nucleic acid sequence or (poly)peptide sequence are defined by a P(N) value that is smaller than 1e-3, preferably smaller than 1e-6, more preferably smaller than 1e-12, even more preferably smaller than 1e-24, most preferably smaller than 1e-48.

The isolated nucleic acid molecules encoding the (poly)peptides of the invention can furthermore be used for gene therapy. The nucleic acid molecule can be introduced at the site of inflammation to act locally or at a distant site. Gene therapy is via viral vectors, such as, but not limited to, adenoviral vectors, adeno-associated viral vectors or lentiviral vectors. Alternatively, non-viral vectors, such as those based on liposomes or polymers may be used. Gene therapeutic strategies are based on (1) *in vivo* gene therapy, where the isolated nucleic acid molecules are introduced into target cells *in vivo,* or (2) *ex vivo* gene therapy, where the isolated nucleic acid molecules are introduced into target cells *ex vivo,* followed by administration of the transduced cells, or a subpopulation of the transduced cells, into an individual. The invention also relates to the vectors for use in gene therapy and to transduced cells.

The (poly)peptides of the invention may be used in a method for treating a subject suffering from inflammation by administering a therapeutically effective amount of a (poly)peptide of the invention and a method for gene therapeutically treating a subject suffering from inflammation by administering a therapeutically effective amount of a nucleic acid molecule, as well as a method for treating a subject suffering from staphylococcus infection by administering a therapeutically effective amount of an antibody and/or biologically active fragment thereof.

The nucleic acid molecules encoding the (poly)peptides of the invention can be used in a method for isolating from an organism a gene encoding a protein having LPI activity, which method comprises screening of a genomic or cDNA library of that organism with a probe based on the nucleic acid molecule, and isolation of the positive clones.

Also disclosed are micro-organisms harboring one or more nucleic acid molecules encoding the (poly)peptides of the invention for use as a medicament for the treatment of acute and chronic inflammation reactions, such as listed in Table 2.

All molecules described and/or claimed herein, i.e. nucleic acid molecules, (poly)peptides, non-(poly)peptides, fragments, derivatives and analogues, may find various other applications. Such applications include, but are not limited to:
- Isolation of factors that can bind the above mentioned molecules. Examples of such factors being receptors and proteins. Such isolation can for instance be performed using the yeast two hybrid system or using tagged molecules of the invention as bait for fishing.
- Making phage display libraries, which can in turn be used for determining active domains, functional equivalents etc.
- Identifying signal transduction pathways that are activated or inactivated by LPI and the molecules of the invention.
- Assay for determination of the biological LPI activity (receptor expression upregulation).

According to this invention peptoids and peptidomimetics can be designed on the basis of the claimed LPI (poly)peptides.

Various definitions for peptidomimetics have been formulated in literature. Among others, peptidomimetics have been described as "chemical structures designed to convert the information contained in peptides into small non-peptide structures", "molecules that mimic the biological activity of peptides but no longer contain any peptide bonds", "structures which serve as appropriate substitutes for peptides in interactions with receptors and enzymes" and as "chemical Trojan horses".

In general, peptidomimetics can be classified into two categories. The first consists of compounds with non-peptide-like structures, often scaffolds onto which pharmacophoric groups have been attached. Thus, they are low molecular-weight compounds and bear no structural resemblance to the native peptides, resulting in an increased stability towards proteolytic enzymes.

The second main class of peptidomimetics consists of compounds of a modular construction comparable to that of (poly)peptides. These compounds can be obtained by modification of either the (poly)peptide side chains or the (poly)peptide backbone. Peptidomimetics of the latter category can be considered to be derived of (poly)peptides by replacement of the amide bond with other moieties. As a result, the compounds are expected to be less sensitive to degradation by proteases. Modification of the amide bond also influences other characteristics such as lipophilicity, hydrogen bonding capacity and conformational flexibility, which in favourable cases may result in an overall improved pharmacological and/or pharmaceutical profile of the compound.

Oligomeric peptidomimetics can in principle be prepared starting from monomeric building blocks in repeating cycles of reaction steps. Therefore, these compounds may be suitable for automated synthesis analogous to the well-established preparation of peptides in peptide synthesizers. Another application of the monomeric building blocks lies in the preparation of peptide/peptidomimetic hybrids, combining natural amino acids and peptidomimetic building blocks to give products in which only some of the amide bonds have been replaced. This may result in compounds which differ sufficiently from the native peptide to obtain an increased biostability, but still possess enough resemblance to the original structure to retain the biological activity.

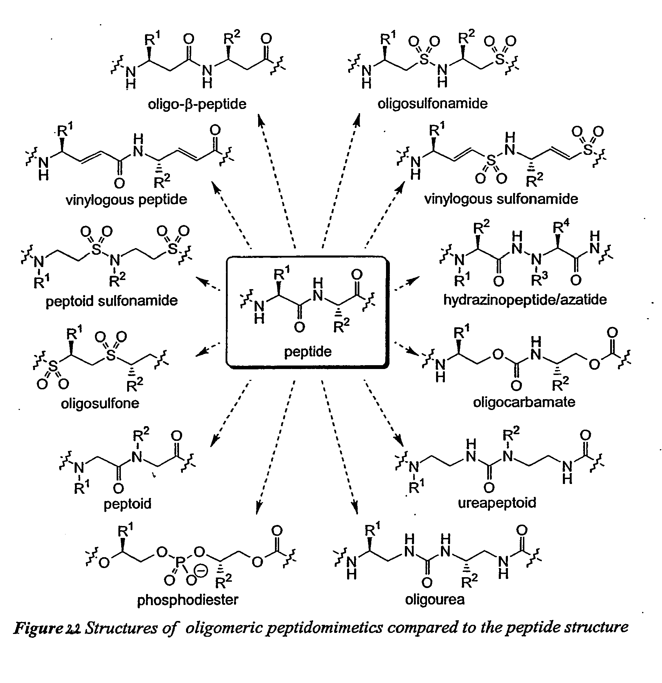

Suitable peptidomimetic building blocks for use in the invention are amide bond surrogates, such as the oligo-ß-peptides (Juaristi, E. Enantioselective Synthesis of b-Amino Acids; Wiley-VCH: New York, 1996**),** vinylogous peptides (Hagihari, M. et al., J. Am. Chem. Soc. 1992, 114, 10672-10674), peptoids (Simon, R.J. et al., Proc. Natl. Acad. Sci. USA 1992, 89, 9367-9371; Zuckermann, R.N. et al., J. Med. Chem. 1994, 37, 2678-2685; Kruijtzer, J.A.W. & Liskamp, R.M.J. Tetrahedron Lett. 1995, 36, 6969-6972); Kruijtzer, J.A.W. Thesis; Utrecht University, 1996**;** Kruijtzer, J.A.W. et al., Chem. Eur. J. 1998, 4, 1570-1580), oligosulfones (Sommerfield, T. & Seebach, D. Angew. Chem., Int. Ed. Eng. 1995, 34, 553-554), phosphodiesters (Lin, P.S.; Ganesan, A. Bioorg. Med. Chem. Lett. 1998, 8, 511-514), oligosulfonamides (Moree, W.J. et al., Tetrahedron Lett. 1991, 32, 409-412; Moree, W.J. et al., Tetrahedron Lett. 1992, 33, 6389-6392; Moree, W.J. et al., Tetrahedron 1993, 49, 1133-1150; Moree, W.J. Thesis; Leiden University, 1994**;** Moree, W.J. et al., J. Org. Chem. 1995, 60, 5157-5169; de Bont, D.B.A. et al., Bioorg. Med. Chem. Lett. 1996, 6, 3035-3040; de Bont, D.B.A. et al., Bioorg. Med. Chem. 1996, 4, 667-672; Löwik, D.W.P.M. Thesis; Utrecht University, 1998**),** peptoid sulfonamides (van Ameijde, J. & Liskamp, R.M.J. Tetrahedron Lett. 2000, 41, 1103-1106), vinylogous sulfonamides (Gennari, C. et al., Eur. J. Org. Chem. 1998, 2437-2449), azatides (or hydrazinopeptides) (Han, H. & Janda, K.D. J. Am. Chem. Soc. 1996, 118, 2539-2544), oligocarbamates (Paikoff, S.J. et al., Tetrahedron Lett. 1996, 37, 5653-5656; Cho, C.Y. et al., Science 1993, 261, 1303-1305), ureapeptoids (Kruijtzer, J.A.W. et al., Tetrahedron Lett. 1997, 38, 5335-5338; Wilson, M.E. & Nowick, J.S. Tetrahedron Lett. 1998, 39, 6613-6616) and oligopyrrolinones (Smith III, A.B. et al., J. Am. Chem. Soc. 1992, 114, 10672-10674). **Figure 22** shows the structures of these peptidomimetic building blocks.

The vinylogous peptides and oligopyrrolinones have been developed in order to be able to form secondary structures (ß-strand conformations) similar to those of peptides, or mimic secondary structures of peptides. All these oligomeric peptidomimetics are expected to be resistant to proteases and can be assembled in high-yielding coupling reactions from optically active monomers (except the peptoids).

Peptidosulfonamides are composed of α- or β-substituted amino ethane sulfonamides containing one or more sulfonamide transition-state isosteres, as an analog of the hydrolysis of the amide bond. Peptide analogs containing a transition-state analog of the hydrolysis of the amide bond have found a widespread use in the development of protease inhibitor e.g. HIV-protease inhibitors.

Another approach to develop oligomeric peptidomimetics is to completely modify the peptide backbone by replacement of all amide bonds by non-hydrolyzable surrogates e.g. carbamate, sulfone, urea and sulfonamide groups. Such oligomeric peptidomimetics may have an increased metabolic stability. Recently, an amide-based alternative oligomeric peptidomimetics has been designed viz. N-substituted Glycine-oligopeptides, the so-called peptoids. Peptoids are characterized by the presence of the amino acid side chain on the amide nitrogen as opposed to being present on the α-C-atom in a peptide, which leads to an increased metabolic stability, as well as removal of the backbone chirality. The absence of the chiral α-C atom can be considered as an advantage because spatial restrictions which are present in peptides do not exist when dealing with peptoids. Furthermore, the space between the side chain and the carbonyl group in a peptoid is identical to that in a peptide. Despite the differences between peptides and peptoids, they have been shown to give rise to biologically active compounds.

Translation of a (poly)peptide chain into a peptoid peptidomimetic may result in either a peptoid (direct-translation) or a retropeptoid (retro-sequence). In the latter category the relative orientation of the carbonyl groups to the side chains is maintained leading to a better resemblance to the parent peptide.

Review articles about peptidomimetics are: Adang, A.E.P. et al.; Recl. Trav. Chim. Pays-Bas 1994, 113, 63-78; Giannis, A. & Kolter, T. Angew. Chem. Int. Ed. Engl. 1993, 32,1244-1267; Moos, W.H. et al., Annu. Rep. Med. Chem. 1993, 28, 315-324; Gallop, M.A. et al., J. Med. Chem. 1994, 37, 1233-1251; Olson, G.L. et al., J. Med. Chem. 1993, 36, 3039-30304; Liskamp, R.M.J. Recl. Trav. Chim. Pays-Bas 1994, 113, 1-19; Liskamp, R.M.J. Angew. Chem. Int. Ed. Engl. 1994, 33, 305-307; Gante, J. Angew. Chem. Int. Ed. Engl. 1994, 33, 1699-1720; Gordon, E.M. et al., Med. Chem. 1994, 37, 1385-1401; and Liskamp, R.M.J. Angew. Chem. Int. Ed. Engl. 1994, 33, 633-636.

The invention thus furthermore relates to molecules that are not LPI (poly)peptides themselves but have a structure and function similar to those of the LPI (poly)peptides described herein. Examples of such molecules are the above described peptidomimetics, but also compounds in which one or more of the amino acids are replaced by non-proteinogenic amino acids or D-amino acids. When reference is made in this application to (poly)peptides, it is intended to include also such other compounds that have a similar or the same structure and function and as a consequence a similar or the same biological LPI activity as the (poly)peptides.

More in particular substitutions can be made with non-proteinogenic amino acids selected from the group consisting of 2-naphtylalanine (Nal(2)), ß-cyclohexylalanine (Cha), p-amino-phenylalanine ((Phe(p-NH₂), p-benzoyl-phenylalanine (Bpa), ornithine (Orn), norleucine (Nle), 4-fluoro-phenylalanine (Phe(p-F)), 4-chloro-phenylalanine (Phe(p-Cl)), 4-bromo-phenylalanine (Phe(p-Br)), 4-iodo-phenylalanine (Phe(p-I)), 4-methyl-phenylalanine (Phe(p-Me)), 4-methoxy-phenylalanine (Tyr(Me)), 4-nitro-phenylalanine (Phe(p-N02)).

Suitable D-amino acids for substituting the amino acids in the (poly)peptides of the invention are for example those that are selected from the group consisting of D-phenylalanine, D-alanine, D-arginine, D-asparagine, D-aspartic acid, D-cysteine, D-glutamic acid, D-glutamine, D-histidine, D-isoleucine, D-leucine, D-lysine, D-methionine, D-proline, D-serine, D-threonine, D-tryptophan, D-tyrosine, D-valine, D-2-naphtylalanine (D-Nal(2)), ß-cyclohexyl-D-alanine (D-Cha), 4-amino-D-phenylalanine (D-Phe(p-NH₂)), p-benzoyl-D-phenylalanine (D-Bpa), D-Ornithine (D-Orn), D-Norleucine (D-Nle), 4-fluoro-D-phenylalanine (D-Phe(p-F)), 4-chloro-D-phenylalanine (D-Phe(p-Cl)), 4-bromo-D-phenylalanine (D-Phe(p-Br)), 4-iodo-D-phenylalanine (D-Phe(p-I)), 4-methyl-D-phenylalanine (D-Phe(p-Me)), 4-methoxy-D-phenylalanine (D-Tyr(Me)), 4-nitro-D-phenylalanine (D-Phe(p-N02)).

One or more of the amino acids in the (poly)peptides can be replaced by peptoid building blocks, e.g. selected from the group consisting of N-substituted glycines, such as N-benzylglycine (NPhe), N-methylglycine (NAla), N-(3-guanidinopropyl)glycine (NArg), N-(Carboxymethyl)glycine (NAsp), N-(carbamylmethyl)glycine (NAsn), N-(thioethyl)-glycine (NhCys), N-(2-carboxyethyl)glycine (NGlu), N-(2-carbamylethyl)glycine (NGln), N-(imidazolylethyl)glycine (NhHis), N-(1-methylpropyl)glycine (NIle), N-(2-methylpropyl)glycine (NLeu), N-(4-aminobutyl)glycine (NLys), N-(2-methylthioethyl)glycine (NMet), N-(hydroxyethyl)glycine (NhSer), N-(2-hydroxypropyl)glycine (NhThr), N-(3-indolylmethyl)glycine (NTrp), N-(p-hydroxyphenmethyl)-glycine (NTyr), N-(1-methylethyl)glycine (NVal).

All compounds of the invention may also be in cyclic form. A cyclic compound may have improved potency, stability, rigidity and/or other pharmaceutical and/or pharmacological characteristics.

All molecules of the invention can be labelled in any way. Examples of labelling include but are not limited to fluorescence, biotin, radioactive labelling etc. Such labelled molecules can be used for screening of compounds that resemble or overlap with the biological activity of LPI, as well as identification of binding sites, both *in vivo* and *in vitro,* and for tracing LPI protein or nucleic acid in an organism.

The present invention will be further illustrated in the examples that follow and that are in no way intended to be limiting to this invention. In this description and the examples reference is made to the following figures and tables:
**Figure 1** illustrates the organization of the genes in the part of the bacteriophage that was called the pathogenicity Island SaPI-5. Shown is SaPI-5 in the 5' region of the bacteriophage of MRSA-16 which is incorporated in the structural gene of ß-toxin. The position and orientation of the four genes of interest: lpi, chp, sak and sea are indicated. Orf1 and orf2 represent structural genes of the bacteriophage.
**Figure 2a** shows the sequence of the LPI gene from *S. aureus* MRSA-16. The Shine Dalgarno sequence (AGGAGA) and the LPI open reading frame (ORF) are underlined. The nucleotides encoding the mature protein are indicated by a double line. The diverging nucleotide of *S. aureus* NCTC 8325 and N315 is indicated above the sequence.
**Figure 2b** shows the sequence of the LPI-B, LPI-C and LPI-D genes. The LPI-B and LPI-C open reading frames are underlined. The diverging nucleotides in different LPI-D sequences is indicated above the sequence.
**Figure 3** shows the amino acid sequence deduced for the LPI, LPI-B, LPI-C and LPI-D genes. The region matching the mature LPI, LPI-B, LPI-C and LPI-D protein is underlined. The diverging amino acids in LPI and LPI-D are indicated above the sequence.
**Figure 4** is a representative image of an SDS-PAGE showing the final purified recombinant LPI (rLPI) obtained from an *E. coli* lysate after affinity chromatography over a Nickel column and cleavage of the Histidine tag by Enterokinase.
**Figure 5** shows the effects of rLPI treatment on the uptake of *S. aureus* by human neutrophils. Labelled bacteria were incubated with human sera or isolated immunoglobulins and neutrophils in the presence or absence of rLPI for 15 minutes. Phagocytosis was measured by flow cytometry and **Figure 5** shows the inhibitory effect of 3 µg/ml rLPI on the uptake of *S. aureus* in human sera.
**Figure 6** illustrates the dose-dependent inhibitory effect of rLPI on bacterial uptake. Phagocytosis was performed in 10% human sera.
In **Figure 7** the lack of effect is illustrated of 8 µg/ml rLPI on the Fcγ-receptors mediated phagocytosis of *S. aureus* by neutrophils, tested by incubating bacteria and neutrophils with purified human immunoglobulins only, thus in the absence of complement.
**Figure 8** shows that there are other molecules then LPI that have LPI activity. His-tagged LPI has the same activity as normal rLPI.
**Figure 9** represents the inhibitory effect of rLPI on the deposition of C3b molecules on the surface of *S*. *aureus*. C3b deposition was performed by incubation of *S. aureus* in 10% human serum in the presence of rLPI in time. Bacteria were washed and the amount of C3b on the surface was detected by specific antibodies labelled with fluorescein. C3b deposition was measured by flow cytometry.
**Figure 10** shows the lack of inhibitory effect of rLPI on classical pathway activation as measured in a haemolysis assay.
**Figure 11** shows the inhibitory effect of rLPI on lectin pathway activation. An ELISA based method on mannan-coated plates with C1q depleted serum and C3b deposition as a read-out were used.
**Figure 12** shows the effect of rLPI on alternative pathway activation. In **figure 12A** the alternative pathway activation was assessed by flow cytometric analysis of deposition of C3b on zymosan using only purified C3, factor D, factor B and properdin (factor P) Figure 12B shows the activity of LPI, LPI-B and LPI-C in an alternative pathway assay, measured in a hemolytic assay using rabbit erythrocytes.
**Figure 13** depicts the binding of LPI to zymosan particles. rLPI was labelled with fluorescein and incubated with zymosan in the presence of human serum at various temperatures. Then fluorescence per particle was measured using flow cytometry.
**Figure 14** depicts the binding of LPI to zymosan particles. rLPI was labelled with fluorescein and incubated with zymosan with and without serum or with Lectin depleted serum (LDS) at various time points. Then fluorescence per particle was measured using flow cytometry.
**Figure 15** Depicts the binding of MBL to zymosan particles in the presence and absence of LPI. MBL was detected by flow cytometry after staining with a specific anti-MBL antibody.
**Figure 16** shows the association between of rLPI and recombinant human MASP-2 as analyzed by size-exclusion chromatography using iodinated rLPI.
**Figure 17** shows the inhibition of rMASP-2 in a specific proteolytic cleavage assay, using a fluorescent substrate by rLPI.
**Figure 18** shows Lectin Pathway-mediated C4b deposition on mannan-coated plates in different amounts of serum and the lack of inhibition by increasing amounts of rLPI.
**Figure 19** shows C4b deposition as a result of cleavage of purified C4 by MBL-MASPs captured on mannan coated plates and the lack of inhibition by LPI compared to that of an anti-MBL antibody (total inhibition).
**Figure 20** shows the inhibitory effect of rLPI on C2 cleavage into C2a and C2b as analysed by Western blotting in serum at various time periods **Figure 20a** shows the blot after staining with anti-C2. **Figure 20b** shows the densitometric analysis of this same blot for the specific C2b band.
**Figure 21** shows the activity of related LPI molecules, LPI-B and LPI-C, in a phagocytosis assay similar to **figure 5****.**
**Figure 22** shows peptidomimetic building blocks.

### EXAMPLES

### EXAMPLE 1

### Identification of LPI as an immunomodulating protein of S. aureus

### 1.1 Introducing Staphylococcus aureus pathogenicity island-5 (SaPI-5)

Recently the inventors' lab described CHIPS, a Chemotaxis Inhibitory Protein of *Staphylococcus aureus.* From data obtained of different *S. aureus* genome sequence projects it became clear that MRSA 16, NCTC 8325, and N315 carry the gene for CHIPS (chp). In each strain chp is located on a different bacteriophage inserted in the structural gene for β-hemolysin (hlb). The chp carrying bacteriophage of MRSA-16 (phi-MRSA-16), bacteriophages of NCTC 8325 (phi-NCTC 8325) and the bacteriophages of N315 (phi-N315) are approximately 45kb and have almost identical 5'regions. The remaining 37kb of phi-MRSA-16, phi-NCTC 8325 and phi-N315 are poorly homologous.

Beside chp the 5' homologous region of the three bacteriophages can contain the genes for the virulence factors: staphylokinase (sak), enterotoxin A (sea) (In N315 sea is replaced by enterotoxin P (sep)). Sak is located just downstream of chp, sea or sep (in N315) is positioned just upstream of sak **(****Figure 1****).** In addition, MSSA and Mu50 carry the bacteriophages phi-MSSA and phi-Mu50 containing a 5' end almost identical to phi-MRSA 16, phi-NCTC 8325, and phi-N315. On the 5'end of phi-MSSA and phi-Mu50, sak and sea were found in the same conformation as the three other bacteriophages, except for a 0.78kb chp cassette that is missing. The remaining 32.1kb of this phage share little similarity to phi-MRSA-16, phi-NCTC 8325 phi-N315 or with each other.

In strain NU3-1 a 4.2 kb fragment was found carrying both chp and sak identical to the 5' region of phi-MRSA-16, phi-NCTC 8325, phi-N315, phi-MSSA and phi-Mu50 yet in this case no sign of a bacteriophage was found (T. Horii et al., FEMS Microbial Letters 185:221-224 (2000)). So the chp and sak-encoding region of NU3-1 is a direct insertion in the genome of *S. aureus.* Based on these data it is believed that chp, sak and sea are located on *Staphylococcus aureus pathogenicity island-5* (SaPI-5). SaPI-5 can contain up to 3 known virulence factors in a strict order **(****Figure 1****).**

### 1.2 SaPI-5 a cluster of staphylococcal immune modulatory' proteins

CHIPS interacts specifically with the C5a receptor (C5aR) as well as the formylated peptide receptor (FPR) of human neutrophils resulting in the specific and total downregulation of the response to both receptors. Staphylokinase (SAK) is described as a thrombolytic factor, by transforming plasminogen into the protease plasmin (M. Parry et al., TIBS 25:53-59 (2000)). The inventors recently found that SAK and plasmin locate on the staphylococcal surface and are there capable of cleaving human IgG at the hinge region, thereby destroying its opsonic features. In addition to that, surface located plasmin also cleaved C3b, again removing opsonic molecules from the surface of staphylococci. Thus also SAK has strong anti-innate immunity and thus anti-inflammatory properties.

Enterotoxins are well known to be superantigenic (M.Dinges et al., Clin. Microbiol. Rev., 13:16-34 (2000)) but recently Enterotoxin A is also described to down-regulate the receptors CCR1, CCR2 and CCR5 on human peripheral blood monocytes resulting in decreased chemokine responsiveness of these cells (R. Rahimpour et al., J. of Imm. 162:2299-2307 1999)).

As mentioned above chp, sak and sea or sep are all located on SaPI-5, which means we are dealing here with a cluster of genes playing an important role in innate immune modulation.

### 1.3 LPI is part of SaPI-5

Beside these three known immune modulators an open reading frame was found on SaPI-5 encoding a putative protein of 116 amino. Analyses revealed we are dealing with an excreted protein since it contains a classical staphylococcus aureus signal peptide. Because of its location on SaPI-5 next to three Staphylococcal immune modulators we speculated on a similar function of this protein.

### RESULTS

### SaPI-5

**Figure 1** shows the organization of SaPI-5 in the 5' region of phi-MRSA-16, which is incorporated in the structural gene of ß-toxin. The position and orientation of LPI, chp, sak and sea are indicated. Orf1 and orf2 represent structural genes of the bacteriophage.

### LPI (Lectin Pathway inhibitor)

The gene was named lpi. It encodes an open reading frame of 348 bp preceded by a reasonable Shine Dalgarno sequence for initiation of translation (J. Shine and L. Dalgarno, Proc. Natl. Acad. Sci. USA, 71:1342-1346 (1974)) and followed by one stop codon (Figure 2a). In Figure 2a, the Shine Dalgarno sequence (AGGAGA) and the LPI open reading frame (ORF) are underlined. The nucleotides encoding the mature protein are indicated by a double line. The diverging nucleotide of *S. aureus* NCTC 8325 and N315 is indicated above the sequence. The sequences of homologous genes lpi-B and lpi-C that are also part of this invention are given in Figure 2b together with the sequence for lpi-D. The LPI-B, LPI-C and LPI-D open reading frames are underlined. Besides these three genes no further significant homology was found with other genes or proteins in the databases to date. The N-terminal 31 amino acids seem to form a signal peptide for secretion across the cytoplasmic membrane (3 positively charged residues followed by a non-charged region of 20 amino acids and an ALA-X-ALA consensus motive for cleavage by the signal peptidase 1 **(****figure 3****)** (G. von Heijne, Nucl. Acids Res. 14:4683-4690 (1986)).

**Figure 3** shows the amino acid sequence deduced for the LPI, LPI-B, LPI-C and LPI-D genes. The region matching the mature LPI, LPI-B, LPI-C or LPI-D protein is underlined. The deduced mature protein LPI has a size of 85 amino acids and 9.8 kDa and an isoelectric point of 9.06 LPI was found in five different *S. aureus* stains. In Mu50 the protein was named BAB58104 and in N315 BAB43028. The sequences were compared and found to be identical with one exception. In LPI of NCTC 8325 and N315 adenine on position 315 is replaced by a thymidine, which leads to amino acid sequence change glutamine on position 80 into leucine **(****figure 2a** and **3****).** In **figure 3** also the sequences of LPI-B, LPI-C and LPI-D are shown. Mature proteins have the following characteristics:
LPI-B: 85 amino acids and 9.9 kDa and an isoelectric point of 9.18
LPI-C: 85 amino acids and 9.9 kDa and an isoelectric point of 8.88
LPI-D: 86 amino acids and 9.9 kDa and an isolelectric point of 9.06.

### EXAMPLE 2

### Cloning, and expression of the LPI-, LPI-B- and LPI-C-encoding genes (lpi, lpi-B and lpi-C) of Staphylococcus aureus

### MATERIAL AND METHOD

### 3.1 Bacterial strains, plasmids and growth conditions

*Staphylococcus aureus* Newman was used as the source of LPI, *Escherichia coli* DH5α was used as a cloning host (F.M. Ausubel et al., Current Protocols in Molecular Biology, John Wiley and Sons, Inc., New York, N.Y. (1990)). All strains were grown in BM broth (1% tryptone, 0.5% yeast extract, 0.5% NaCl, 0.1% K₂HPO₄, 0.1% glucose) at 37°C unless otherwise noted.

### 3.2 Production of recombinant polypeptides having LPI activity in E.coli

The production method used to produced LPI in *E. coli* can also be used for other (poly)peptides having LPI activity. This production method is illustrated herein below.

The DNA sequence for LPI, LPI-B or LPI-C from *S. aureus* is cloned into a suitable vector that enables efficient expression of LPI in competent *E. coli* host cells using conventional molecular biology techniques. The strategy used enables expression of the complete LPI or LPI-like protein linked to a removable HIS-tag at the N-terminus in the cytoplasm of *E.coli.* The T7 Expression System (pRSET B vector; Invitrogen) was used that enables expression of non-toxic proteins in *E.coli.* This system uses the strong phage T7 promotor for high-level, regulated expression in any *E.coli* strain with a multicloning vector. The vector contains an N-terminal polyhistidine (6xHis) tag for rapid purification, an Xpress epitope for easy detection with an anti-Xpress antibody and an Enterokinase cleavage site for removal of fusion tag. The Enterokinase recognition site of the pRSET-B vector was changed from GAC GAT GAC GAT AAG into GAC GAT GAC GAC AAG so that blunt end digestion by PshA1 (Westburg BV, Leusden, The Netherlands) was possible. An extra guanine at the 5' end of the LPI primers was used to complement the enterokinase cleavage site.

*S. aureus* Newman chromosomal DNA was used as template for the PCR reaction using the PƒuTurbo DNA polymerase (Stratagene) that results in a blunt ended PCR product. The primers used are LPI-5' (gagcacaagcttgccaacatcg) (an extra guanine followed with exactly the first amino acid of LPI) and LPI-3' (ccggaattcttaatatttactttttagtgc) (containing the autologous stop codon and a EcoRI-site).

The same procedure was performed for the LPI-B gene from *S. aureus* Newman chromosomal DNA. This fragment is 255 bp, the primers used are lpi-B-5' (gagtagtctggacaaatattt) and lpi-B-3' (ccggaattcttatctatttataatttcat).

The same procedure was performed for the LPI-C gene from chromosomal DNA of an *S. aureus* clinical isolate, positive for lpi-C. This fragment is 255 bp, the primers used are lpi-C-5' (GAGTAGTAAGAAAGACTATAT) and lpi-C-3' (GGAATTCCTTATCTATTTATAATTTCA).

The PCR product is digested with EcoRI and the pRSET B vector with PshA1 to create a blunt end. Thereafter the vector is digested with EcoRI and ligated with the digested PCR product.

For transformation of the vector, Rosetta-gami(DE3) pLysS *E*. *coli* competent cells (Novagen) were used. Clones are screened on carbenicillin, chloramphenicol, kanamycine and tetracycline (Sigma) containing plates and proper ligation of *lpi* is verified by sequencing of the isolated plasmid.

After expression of the LPI, LPI-B or LPI-C gene, the *E. coli* bacteria are lysed and the protein mixture is applied onto His-Trap resin columns (Amersham Biosciences). For that a culture in LB medium is initiated with 1 mM IPTG for 2 h at 37°C. Bacteria are centrifuged and the pellet resuspended in guanidine lysis buffer (6 M guanidine hydrochloride, 20 mM sodium phosphate, 500 mM sodium chloride; pH 7.8), incubated at room temperature for 15 min with rocking, and was then sonicated three times at high intensity for 5 s on ice. After removal of the insoluble debris by centrifugation, lysates were applied to a pre-equilibrated His-Trap resin column.

The column was loaded with 0.1 M nickel sulfate solution and equilibrated with denaturing binding buffer (8 M ureum, 20 mM sodium phosphate, 500 mM sodium chloride; pH 7.8). After application of the lysate, the column was washed with ureum binding buffer with decreasing pH (pH 7.8, pH 6.0 and pH 5.3). Column-bound proteins were brought into native phosphate buffer by changing ureum binding buffer (pH 5.3) into native buffer (200 mM sodium dihydrogen phosphate dihydrate, 5 M sodium chloride, 50 mM sodium phosphate dibasic; pH 5.3) solution. Finally, proteins were eluted in 0.05 M EDTA.

The HIS-tag is removed by enterokinase cleavage followed by removal of the protease with an EK-Away enterokinase preparation. Therefor the eluate is dialyzed overnight in cold digestion buffer (50 mM Tris-HCl, 1 mM CaCl2 and 0.1% Tween-20, pH 8.0), filtered through a 0.45 µm filter and digested with 0.175 µl Enterokinase/ml HIS-LPI product. This amount of Enterokinase is batch-dependent and results in a partial digestion to avoid the generation of breakdown products. The digested product is dialyzed against phosphate buffer pH 7.8 and passed over a fresh Nickel column to eliminate His tags and uncleaved His-tagged LPI (HIS-LPI); the run through is pure recombinant LPI (rLPI). Undigested HIS-LPI can be eluted again from Nickel column for a second digestion round. The Nickel column is finally washed with 50 mM EDTA, 0.5 M NaOH, water, 5 mg/ml NiCl₂, water and stored in 20% ethanol.

All steps in the isolation and digestion of HIS-LPI are checked by SDS-PAGE on a 16.5% Tris-Tricine Ready gel (BioRad). Samples are mixed 1:1 with sample buffer (200 mM Tris-HCl pH 6.8, 2% SDS, 40% glycerol, 0.04% Coomassie), boiled for 5 min and loaded on the gel.

The HIS-tag of the expressed protein contains an X-press epitope that enables detection of the HIS-LPI product by Western blot using the anti-X-press antibody (Invitrogen). Proteins are transferred to a nitrocellulose membrane, blocked with 4% gelatin in PBS and probed with the antibody and the appropriate secondary peroxidase labelled conjugate (Harlow & Lane, 1988, Antibodies: a laboratory manual, Cold Spring Harbor Laboratory). The exact same procedure was followed for His-tagged LPI-B and LPI-C.

### RESULTS

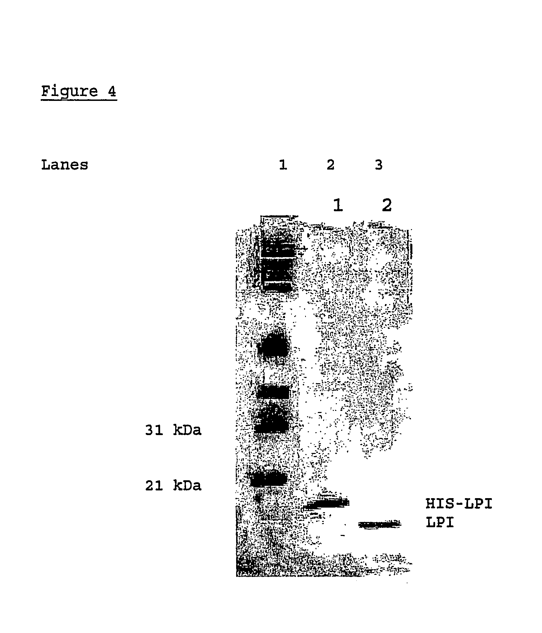

**Figure 4** is a representative image of an SDS-PAGE showing the purified recombinant LPI (rLPI). The first lane (1) shows the complete recombinant product that is encoded by the vector generating the LPI protein with an additional Histidine tag and enterokinase cleavage site. This encodes for a protein with an apparent molecular weight of 13 kDa, while purified enterokinase treated LPI (lane 2) runs at an apparent molecular weight of 10 kDa. Preparations of HIS-tagged and cleaved LPI-B and LPI-C were equally pure.

### EXAMPLE 3

Phagocytosis is an important immunological process that is performed by human phagocytes to eliminate invading bacteria. The following sequential steps can be discriminated, opsonization/recognition/binding, then ingestion of the bacterium by the phagocyte and finally killing and degradation of the bacterium and its toxic compounds.

### 3.1 Phagocytosis

Phagocytosis is a result of recognition of a foreign particle by specific receptors on the plasma membrane of phagocytes. Neutrophils express receptors for serum-derived opsonins, including IgG and opsonic fragments of the complement component C3 (C3b and C3bi). In this example phagocytosis of *S. aureus* by human neutrophils in the presence of human serum was assayed. Moreover, human IgG fractions were isolated from healthy donors and pure IgG molecules used to study Fcγ-receptor mediated (=IgG-mediated) phagocytosis. Recombinant LPI was added to these assays to gain insight in the role of LPI as an immuno-modulating molecule.

### MATERIALS AND METHODS

An overnight culture of bacteria was grown an additional 4 hours in fresh medium. Bacteria were washed and incubated for 1 hour at 37°C with 100µg/ml FITC in 0.1 M carbonate buffer, pH9.6. Free FITC was removed by washing bacteria 2 times. Human neutrophils were purified on a Ficoll/Histopaque gradient as described previously (Troelstra et al., J. Leukocyte Biol. 61, 173-178 (1997)) using heparinized whole blood from a single donor. Human sera were obtained by pooling sera of 10 healthy donors. A total of 50 µl (1.3x107 cfu/ml) of labelled bacteria were incubated with 100 µl human serum, 50 µl rLPI (various concentrations) and 50 µl of 1x10⁶ neutrophils.

Bacteria were mixed with neutrophils in a ratio of 10:1. Phagocytosis was allowed for 15 minutes at 37°C and stopped by fixing samples in 100 ml of 1% paraformaldehyde. Samples were evaluated by flow cytometry.

### RESULTS

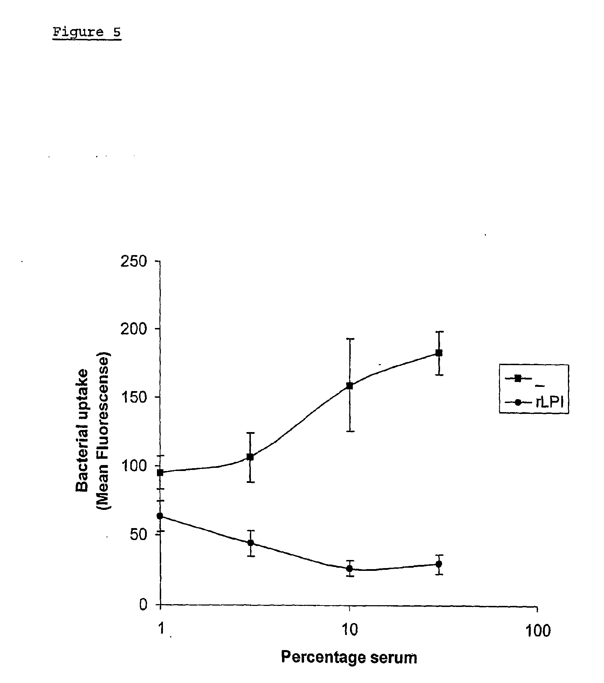

**Figure 5** shows the inhibitory effect of 3 µg/ml rLPI on the uptake of *S. aureus* in normal human serum. Labelled bacteria were incubated with increasing concentrations of human serum in the presence or absence of rLPI. Phagocytosis by human neutrophils was depicted as the mean bacterial uptake by neutrophils. The uptake of labelled bacteria by human neutrophils increases with higher serum concentrations. This figure shows a strong inhibitory effect of rLPI on bacterial uptake.

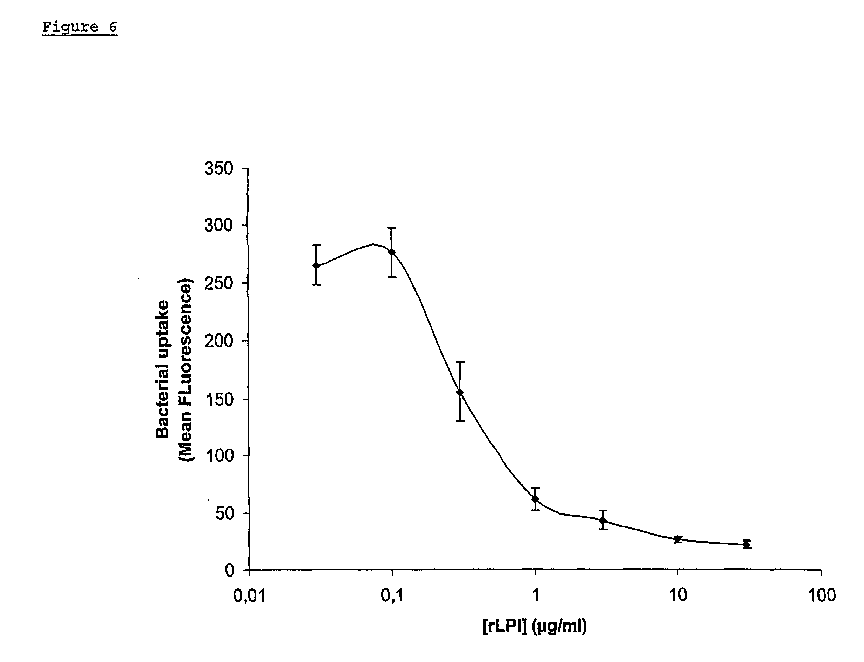

**Figure 6** illustrates the dose-dependent inhibitory effect of rLPI on bacterial uptake. In order to get insight in the effective inhibitory concentrations of rLPI, phagocytosis of labelled *S. aureus* was performed in 10% human serum and with increasing concentrations of rLPI. This figure shows that rLPI has a half-maximum inhibitory concentration (IC50) of 0.3 µg/ml.

### 3.2 IgG-mediated phagocytosis

### MATERIALS AND METHODS

In order to pinpoint whether the observed anti-phagocytic effects of rLPI were mediated by inhibition of complement- or Fcγ-receptor mediated uptake, human sera were replaced for purified IgG during phagocytosis. Human IgG was purified from serum of a healthy volunteer by protein G-affinity chromatography (Amersham Biosciences, Upsalla, Sweden, according to manufacturers instructions) as previously described by Troelstra et al. (Troelstra et al., J. Leukocyte Biol. 61, 173-178 (1997)). IgG was added in various concentrations. Recombinant LPI was tested in a concentration of 8 µg/ml. Phagocytosis was performed for 15 minutes at 37°C. Bacteria were mixed with neutrophils in a ratio of 15:1. Samples were evaluated by flow cytometry and phagocytosis was depicted as the mean bacterial uptake by neutrophils.

### RESULTS

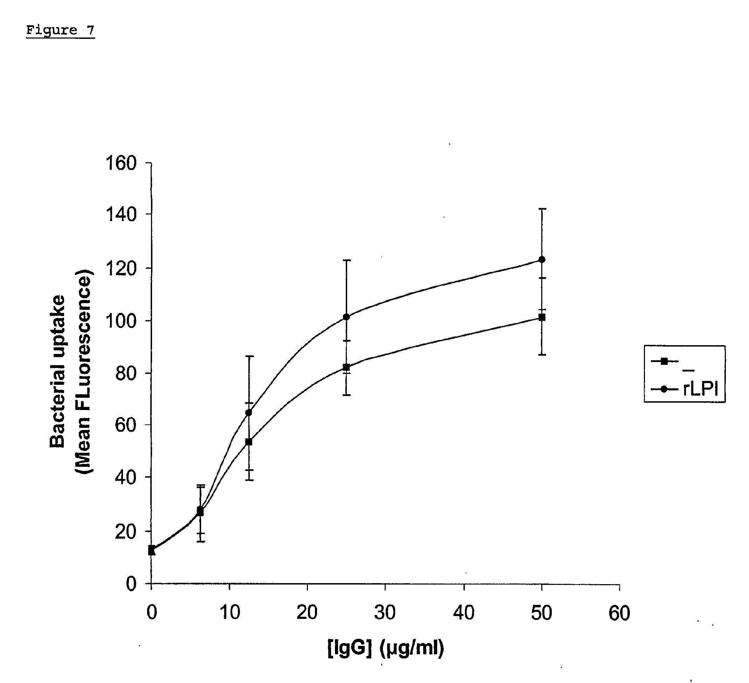

**Figure 7** shows no effect of 8µg/ml rLPI on the phagocytosis of *S.* aureus when this was solely mediated by Fcγ-receptors on neutrophils. This was studied by incubating bacteria and neutrophils with purified human immunoglobulins instead of serum.

3.3 In a similar phagocytosis experiment rLPI was compared to the larger N-terminally extended His-tagged LPI molecule.

### RESULTS

**Figure 8** depicts the inhibitory effect of both rLPI and rHIS-LPI. Both molecules show comparable inhibition at equal concentrations. It was concluded that the presence of a Histidine Tag at the N-terminus of the protein does not interfere with LPI activity.

### EXAMPLE 4

### 4.1 C3b deposition on S. aureus

Activation of complement leads to deposition of C3b and C3bi molecules at the bacterium that are recognized by complement receptors on phagocytes. To study whether LPI is an inhibitor of complement activation an assay was developed to measure C3b deposition at the surface of *S. aureus.*

### MATERIALS AND METHODS

A stationary growth culture of Cowan EMS was washed three times in PBS. 100 ml of 3x10⁷ bacteria/ml were incubated in 10% human sera at 37°C. Bacteria were washed and surface-bound C3b was detected by incubating bacteria with Fluorescein- conjugated Goat F(ab')₂ anti-human C3 (Protos immunoresearch, Diessen, The Netherlands) reacting with intact C3b molecules. Then fluorescence per particle was measured using flow cytometry.

### RESULTS

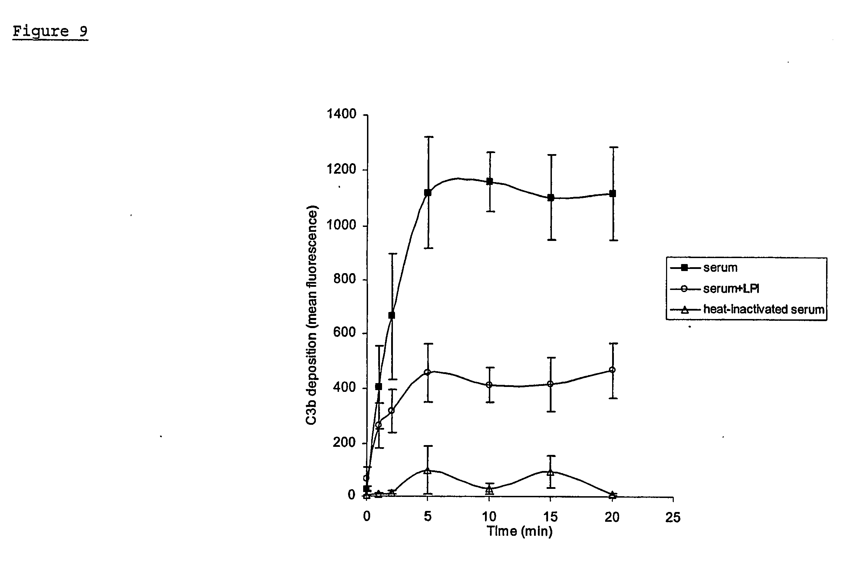

**Figure 9** presents the inhibitory effect of 3 µg/ml rLPI on the deposition of C3b on the surface of *S. aureus.* C3b deposition was performed by incubation of *S. aureus* in 10% human serum in the presence of rLPI for varying time intervals. Bacteria were washed and the amount of C3b on the surface was detected by specific labelled antibodies. C3b deposition is depicted as the mean bacterial fluorescence. Heat-inactivated serum, where complement activity is destroyed, was used to control for antibody specificity.

### EXAMPLE 5

### Complement activation by different pathways

Activation of complement on microorganisms can be initiated via three pathways: the classical, lectin and alternative pathway. In order to recognize all the different micro-organisms we encounter, these pathways act together to activate complement. To study whether the complement-inhibitory role of LPI was due to inhibition of (one of) these three pathways, LPI was tested in 3 well-described assays that specifically measure these pathways

### 5.1 The Classical Pathway (CP)

The CP is activated when C1q binds to antigen-antibody complexes. C1q circulates in serum with two attached serine proteases, C1r and C1s. Upon binding of the C1 complex to an antibody, C1r and C1s are activated. C1s cleaves complement C2 and C4 generating a C4b2a complex that serves as the C3 convertase.

### MATERIALS AND METHODS

To measure the classical pathway activation route, CH50 measurements were performed similar to Klerx et al. (Klerx et al., J Immunol Methods. 1983, 63:215-20). In short, sheep erythrocytes were incubated with anti-sheep erythrocyte antibodies. Then Ab-covered erythrocytes were incubated in human serum for 1 hour at 37°C. Classical pathway mediated complement activation will lead to formation of membrane attack complexes that lyse the erythrocytes. 10 µg/ml of rLPI were added to test the role of LPI in the classical pathway.

### RESULTS

**Figure 10** shows the lack of effect of LPI on classical pathway complement activation as determined by a hemolysis assay with immunoglobulins coated erythrocytes. LPI does not inhibit the classical pathway.

### 5.2 The lectin pathway

The lectin pathway is initiated when MBL or ficolins recognize sugars on microbial surfaces. MBL and ficolin are also complexed with serine proteases called MASPs. MASP-2 is responsible for cleaving C2 and C4 to generate a C3 convertase.

### MATERIALS AND METHODS

Lectin pathway-mediated C3 deposition was performed on mannan coated plates as described by Roos et al. (Roos et al., Molecular Immunology Volume 39, Issue 11, January 2003, Pages 655-668). Briefly, C1q-depleted serum was prepared using serum of a healthy donor and a rabbit IgG anti-human C1q coupled to Biogel A5. Clq-depleted serum was incubated on mannan, an efficient activator of MBL-MASPs, for 1 hour at 37°C. C3b deposition was measured by digoxigenin-conjugated anti-human C3 followed by HRP-conjugated sheep anti-dig antibodies.

### RESULTS

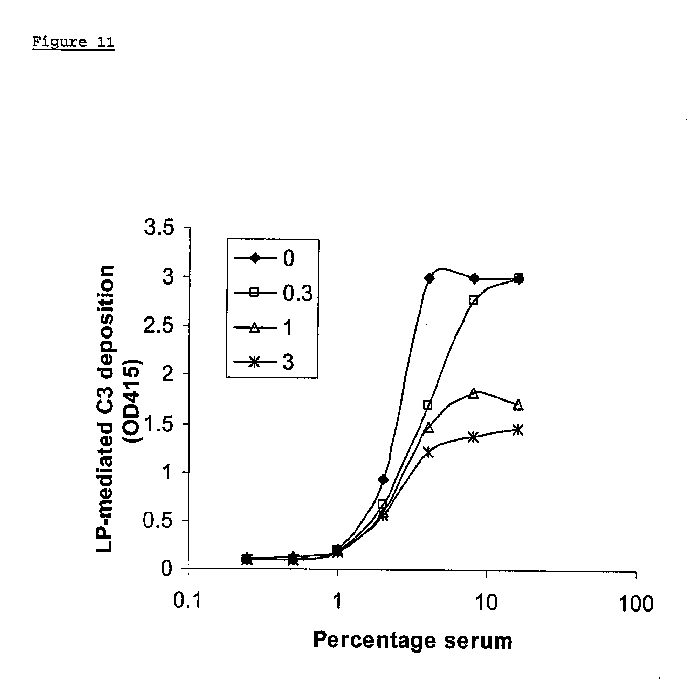

**Figure 11** shows the inhibitory effect of rLPI on lectin pathway activation using an ELISA based method on mannan-coated plates and C1q depleted serum with C3b deposition as a readout. LPI strongly inhibits the lectin pathway of complement activation.

### 5.3 The alternative pathway

### MATERIALS AND METHODS

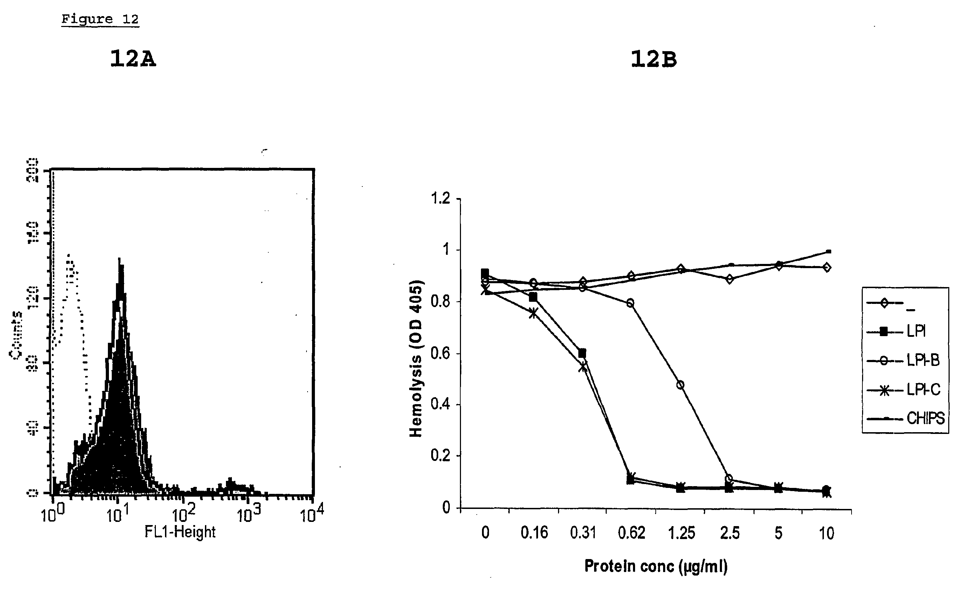

To test the effect of LPI on the alternative pathway, two assays were used. In **figure 12A****,** a method was used as described by Schreiber et al. (Schreiber et al, Proc. Natl. Acad. Sci. USA, Vol. 75, No. 8, pp. 3948-3954).

In short, the complement components C3, factor D, factor B and properdin (factor P) were purified to homogeneity. Then 0.5 mg zymosan was incubated with these purified proteins in concentrations similar to that in 25% serum. After 20, 30 or 40 minutes at 37°C, C3b deposition was evaluated by flow cytometry as described in 4.1.

In **figure 12B** an alternative pathway hemolysis assay was used as previously described by Klerx et al. (*supra*)*.* Briefly, rabbit erythrocytes were suspended in EGTA-VB and incubated with human serum in the presence or absence of recombinant proteins for one hour at 37°C. Erythrocytes were pelleted and 50 µl of supernatant was lysed in 100 µl of water to monitor percentage of haemolysis.

### RESULTS

**Figure 12A** shows the lack of inhibition of rLPI on C3b deposition by flow cytometric analysis of zymosan using only purified C3, factor D, factor B and properdin (factor P). **Figure 12B** shows that the hemolytic assay is inhibited by LPI, LPI-B and LPI-C (CHIPS is the negative control protein here). With purified components we see no influence of LPI on the alternative pathway.

### EXAMPLE 6

### 6.1 LPI-binding

The above-described results have suggested a role for LPI in preventing C3b deposition and subsequent phagocytosis by inhibition of lectin-mediated complement activation. LPI binding experiments on *S. aureus* were performed to get more information about how LPI might work as an inhibitor of complement activation.

### MATERIALS AND METHODS

Recombinant LPI was FITC-labelled by incubating 400 µg/ml rLPI in a 0.1 M sodium carbonate buffer (pH9.6). FITC-labelled rLPI was purified from free FITC using a Fast Desalting column (Amersham Biosciences, Upsalla Sweden). 0.5 mg of washed zymosan incubated in 0% or 10% human sera at 0°C and at 37°C. Also, human sera were depleted from polysaccharide-binding molecules by incubation of 500 ml serum with staphylococcal cell wall homogenate. LPI binding on zymosan in the presence of this lectin-depleted serum was compared with the binding to normal serum at 37°C. After incubation in serum, bacteria were washed and surface-bound rLPI-FITC was measured by flow cytometry.

### RESULTS

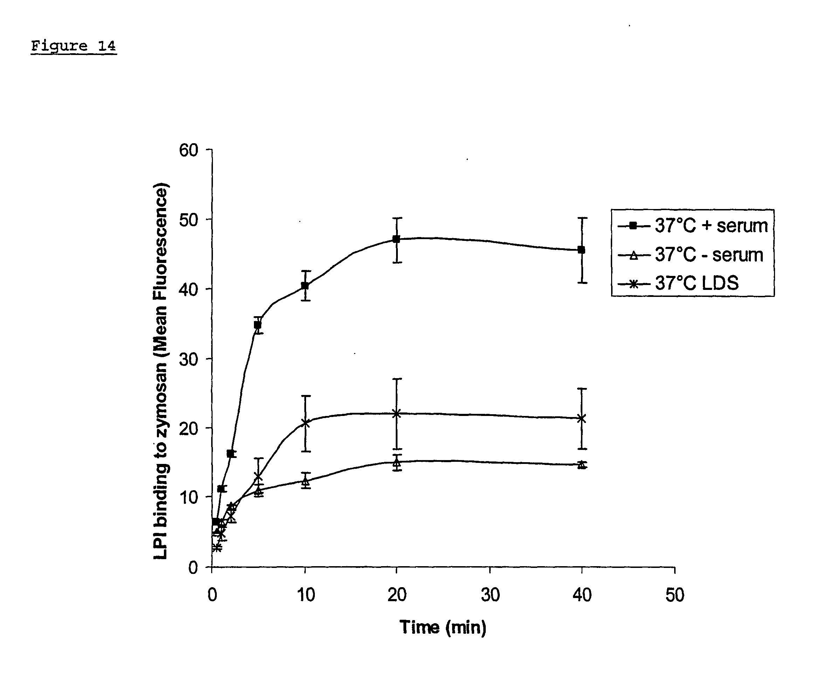

**Figure 13** shows a dose dependent association of LPI-FITC to zymosan particles in flow cytometry. The binding is dependent both on the presence of serum and does not proceed at 0°C. **Figure 14** presents the binding kinetics of rLPI on zymosan. Recombinant LPI-FITC binding is dependent on the presence of human serum, because rLPI does not bind bacteria alone. Moreover, rLPI does not bind to bacteria at 0°C. An activation process seems to be necessary. Compared to rLPI binding in human serum, this binding is completely abolished in lectin-depleted serum. This latter result again demonstrates that LPI affects the lectin route of complement activation and not the classical pathway. rLPI-FITC binding is depicted as the mean bacterial fluorescence. From this it is likely that LPI needs a serum component for its interaction and that this component is activation dependent. Because this component is part of the lectin pathway MASP-2 is the most likely candidate.

### EXAMPLE 7

### What Lectin pathway component is inhibited by LPI? 7.1 MBL

The first component in the lectin pathway is MBL. To evaluate the effects of LPI on MBL binding we analyzed the binding of MBL to Zymosan in the presence or absence of rLPI. **MATERIALS AND METHODS**

0.5 mg of washed zymosan was incubated with 30% of normal human serum or in C3-deficient serum (Sigma) for 30 minutes at 0°C or 37°C respectively. MBL was detected by subsequent incubation of zymosan particles with monoclonal anti-MBL antibodies (Hbt, Uden, The Netherlands) followed by Fluorescein conjugated goat-anti-mouse antibodies. Fluorescence per particle was determined by flow cytometric analysis.

### RESULTS

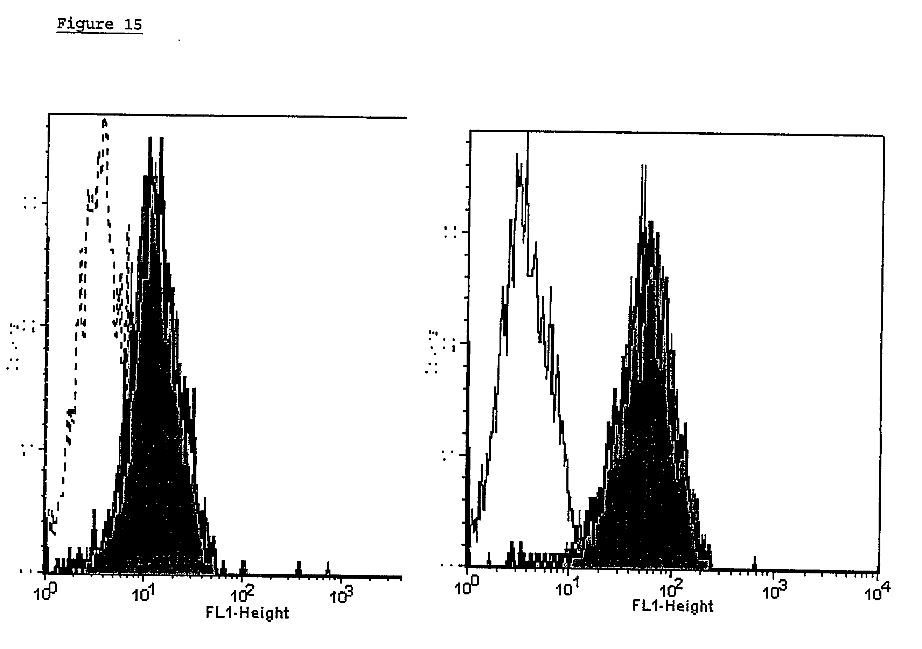

**Figure 15** depicts the binding of MBL to zymosan particles in the presence and absence of LPI. MBL was detected by flow cytometry after staining with a specific anti-MBL antibody. MBL association is not affected by the presence of LPI.

The next step in the lectin pathway involves the MBL associated serine protease-2 (MASP-2). The interaction of LPI with MASP-2 was evaluated in two ways: association and functional inhibition.

### 7.2 MASP-2 association

### MATERIAL AND METHODS

rLPI was labelled with ¹²⁵I using Iodogen (1,3,4,6-tetrachloro-3a,6a-diphenylglycoluril) (Sigma) as the oxidising agent (Fraker and Speck, 1978). Free 125-iodide was removed by desalting on a PD-10 Sephadex G-25 gel filtration column (Pharmacia) which was presaturated with 0.1% (v/v) Emulphogene BC720 [Sigma] in HBS. Radiolabelled protein fractions were pooled and stored at 4°C. ¹²⁵I-LPI was incubated with purified complement proteins, including recombinant MASP-1 and MASP-2 (consisting of the two complement control regions and the serine protease domains as described by Ambrus et al., 2003. G.. J. Immunol. 170 (2003), pp. 1374-1382) for 15 minutes at 37°C. Protein mixtures were run on a Superose-6 column (Amersham) and specific activity of ¹²⁵I-LPI was determined by measuring collected fractions on a Mini-Assay type 6-20 manual g counter (Mini Instruments, Burnham-on-Crouch, Essex, UK).

### RESULTS

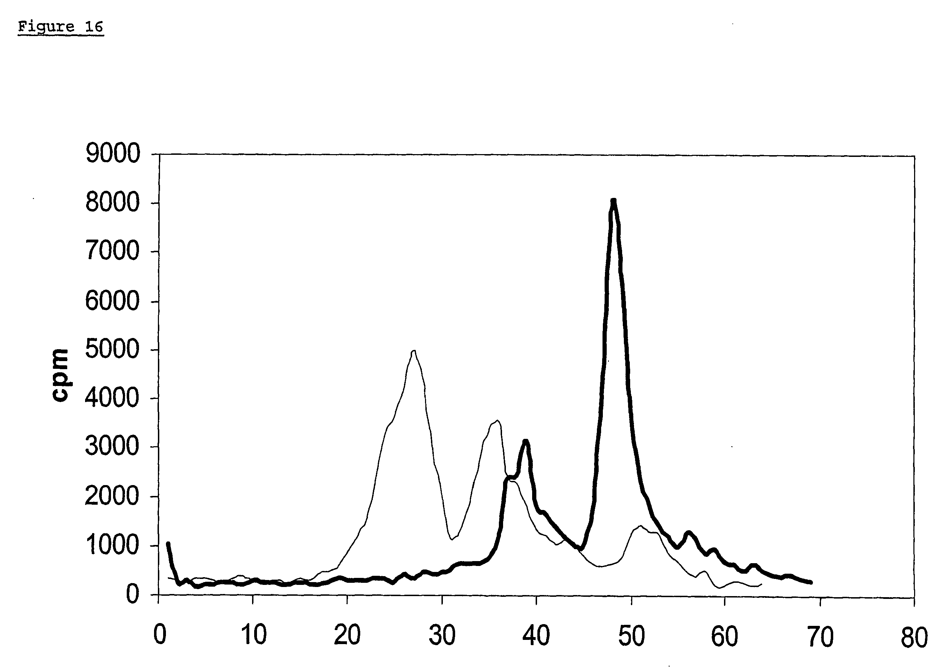

**Figure 16** shows the association between of rLPI and rMASP-2 as analyzed by size-exclusion chromatography using iodinated rLPI. LPI and MASP-2 do interact to form a larger sized molecular complex.

### 7.3 MASP-2 activity

### MATERIAL AND METHODS

0.2 mg rMASP-2 was incubated with 0.1 mg Prothrombin (Calbiochem) and 0.1 mM VPR-AMC (Bachem, Bubendorf, Switzerland) in the presence or absence of various concentrations of rLPI. rMASP-2 will cleave Prothrombin into thrombin. Cleavage of the thrombin specific substrate VPR-AMC was measured every 30 seconds for 1 hour using a microtitre plate reader (Fluoroskan, Thermo Life Sciences, Basingstoke, UK) exciting samples at 355 nm and reading emission at 460 nm.

### RESULTS

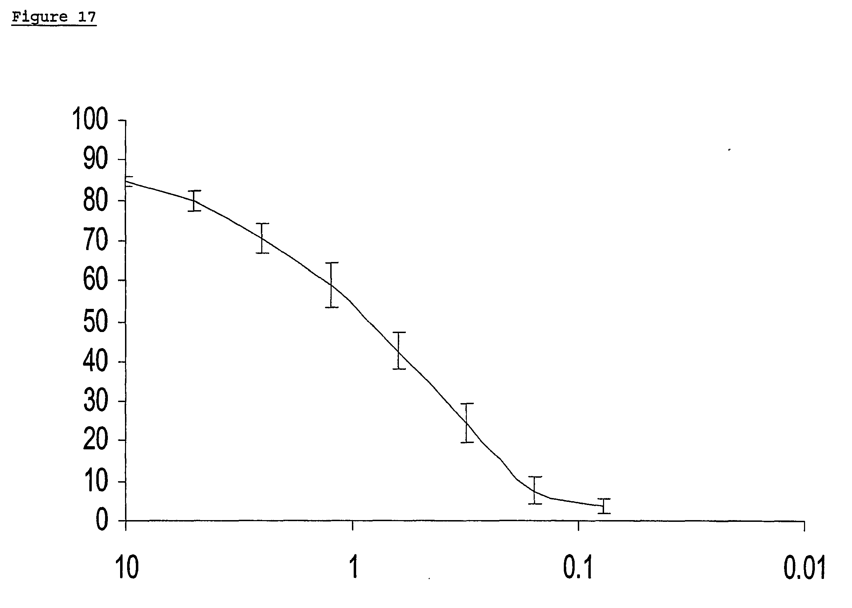

**Figure 17** shows the inhibition of rMASP-2 activity by rLPI, in a specific proteolytic cleavage assay, using a fluorescent substrate. LPI does inhibit MASP-2 activity.

### EXAMPLE 8

### 8.1 The molecular mechanism of LPI

MASP-2 has two specific proteolytic activities: the cleavage of C2 and the cleavage of C4. To determine what the exact mechanism of action of LPI is both actions of MASP-2 were tested and the effects of LPI on these separate steps evaluated.

### MATERIALS AND METHODS

### C4 deposition

LP-mediated C4 deposition was measured exactly as described in section 5.2 for measuring LP-mediated C3 deposition with the exception that a monoclonal antibody against C4 was used in order to detect C4b deposition. Alternatively, MBL-MASP complexes were captured on mannan coated plates by incubating 100 µl of 50 % human serum in 1 M NaCl buffer (to prevent C1q binding to IgG). After washing MBL-MASP complex in low salt buffers, purified human C4 was added and MBL-MASP mediated cleavage of C4 was allowed for 1 hour at 37°C. Deposited C4b molecules were detected as described above.

### C2 cleavage assay

Activation of C2 as a result of incubating human serum with zymosan particles was assayed as following. 0.25 mg of zymosan was incubated with 40% human serum for 0, 20 or 40 minutes. Cleaved and uncleaved C2 molecules were detected by subjecting 10% of the incubated serum to gel electrophoresis. Western blotting using goat anti-human C2 (Quidel, San Diego, CA) followed by HRP conjugated Donkey anti-goat antibodies and ECL (Amersham).

### RESULTS

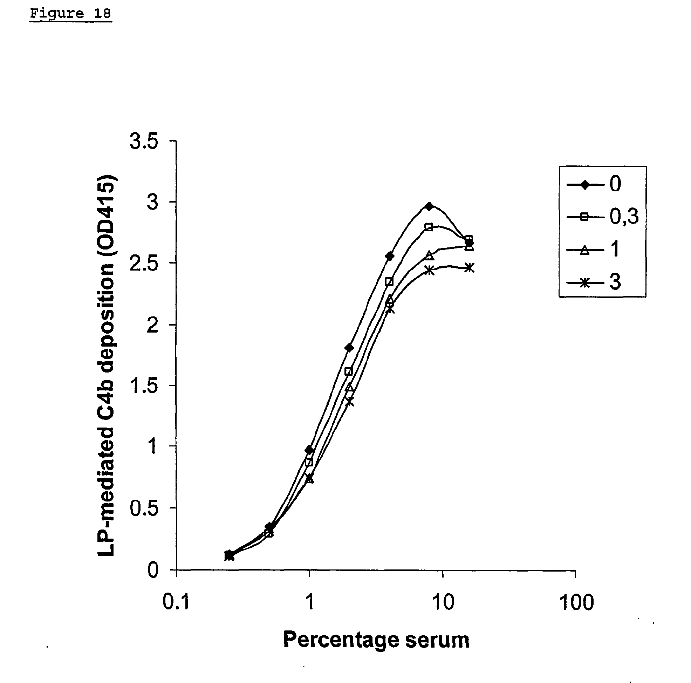

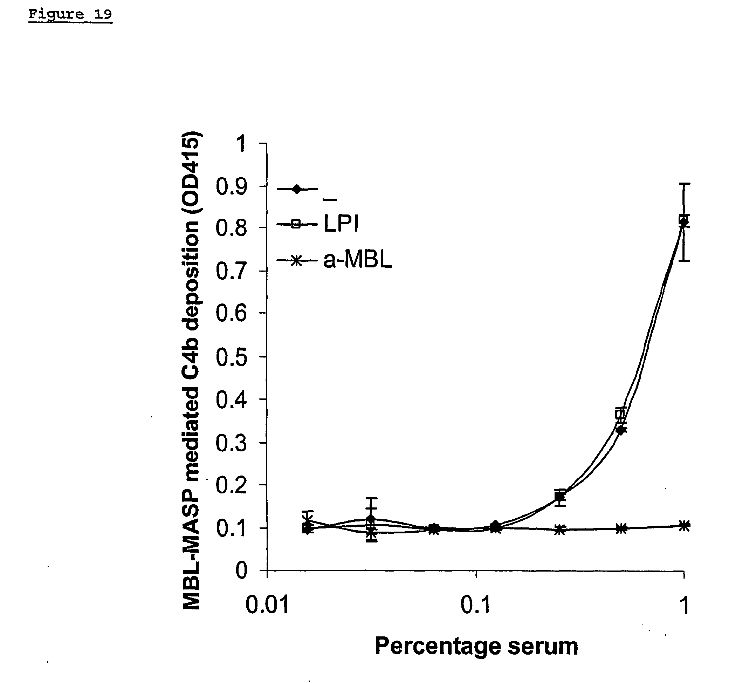

**Figure 18** shows LP-mediated C4b deposition on mannan-coated plates in the presence of increasing amounts of rLPI and different amounts of serum. No inhibition is observed. In Figure 19 C4 activation by captured MBL-MASP complexes cleaving purified human C4 is depicted. The effect of the addition of LPI is compared to that of an anti-MBL antibody (total inhibition). LPI shows no inhibition of MASP-2 dependent C4 deposition. The C2 cleavage however is clearly affected by LPI.

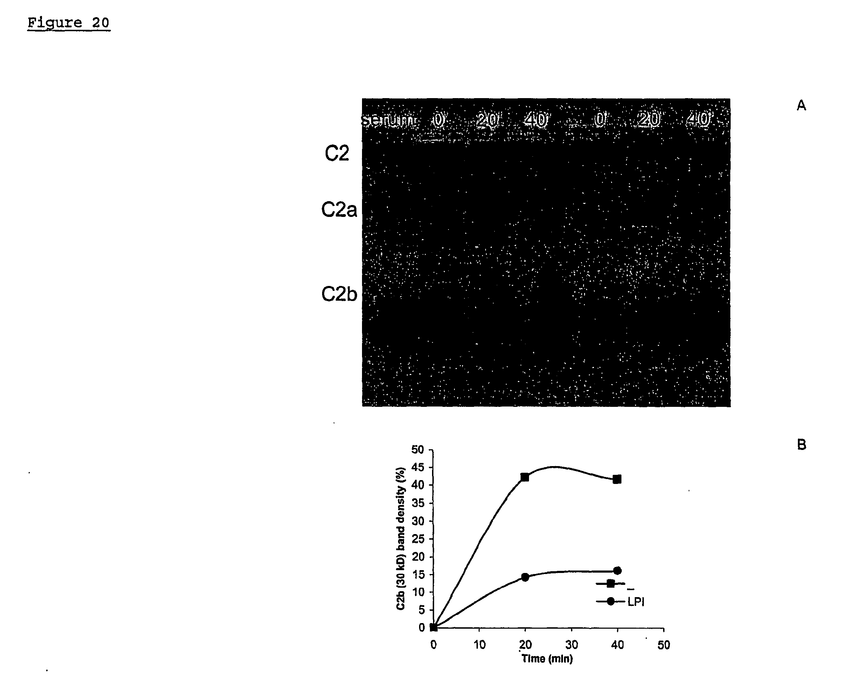

In **Figure 20** the C2 cleavage into C2a and C2b was analysed by Western blotting after complement activation had preceded in serum at various time periods with or without LPI. **Figure 20a** shows the blot after staining with anti-C2. **Figure 20b** shows the densitometric analysis of this same blot for the specific C2b band. LPI clearly inhibits C2 cleavage.

Taken together LPI is designed to interfere with MASP-2 mediated cleavage of C2, thereby blocking the entire lectin pathway (MBL and ficolins are all dependent on MASP-2). Furthermore, this action makes LPI also completely specific for the lectin pathway, without interfering with other complement pathways.

### EXAMPLE 9

### The LPI activity of LPI-like molecules LPI-B and LPI-C

### 9.1. In a separate phagocytosis experiment LPI-B and LPI-C were tested for LPI-activity

### MATERIALS AND METHODS

The phagocytosis assay was performed as described in 3.1.

### RESULTS

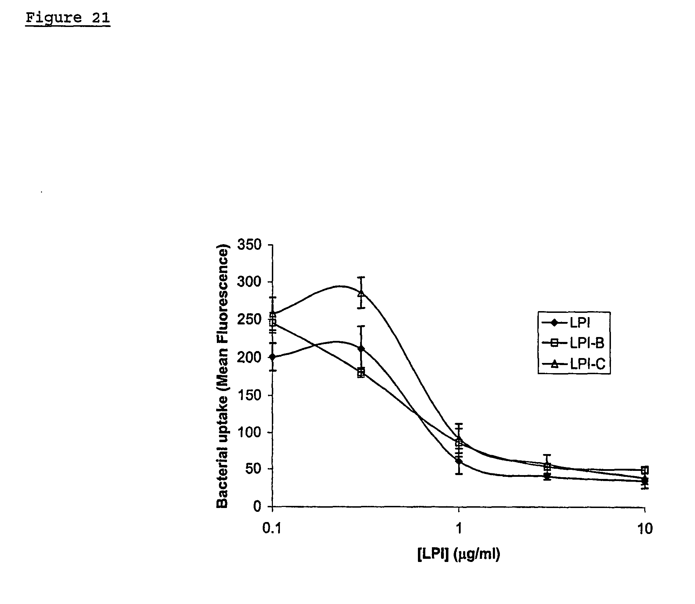

**Figure 21** depicts the anti-phagocytic effect of both rLPI-B and LPI-C in the presence of different concentrations of serum. From this we conclude that LPI-B and LPI-C have at least LPI activity.

## Claims

1. Peptide or polypeptide encoded by a nucleotide sequence corresponding to a sequence being selected from the group consisting of:
a) a nucleotide sequence comprising a part of one of the sequences as depicted in **Figure 2a** and **2b** and identified as **SEQ ID NO:2,; SEQ ID NO:4; SEQ ID NO:6**;
b) nucleotide sequences encoding a peptide or polypeptide having the amino acid sequence depicted in **Figure 3** and identified as **SEQ ID NO:3, SEQ ID NO:5 or SEQ ID NO:7;**
c) nucleotide sequences encoding a peptide or polypeptide having a portion of the amino acid sequence depicted in **Figure 3** identified as **SEQ ID NO:3, SEQ ID NO:5** or **SEQ ID NO:7;**
d) nucleotide sequences being at least 40% identical to any one of the nucleotide sequences a), b) or c);
e) nucleotide sequences hybridizing at stringent conditions with any one of the nucleotide sequences a), b), c) or d), and
f) nucleotide sequences complementary to any of the nucleotide sequences a), b), c), d) or e),
wherein said peptide or polypeptide has LPI (Lectin Pathway Inhibitor) activity which prevents activation of the lectin pathway of complement activation by specifically preventing the MASP-2 Mannose Binding Lectin Associated Serine Protease-2) dependent cleavage of C2 into C2a and C2b, and said peptide or polypeptide is for use in prophylaxis or treatment of acute and chronic inflammation reaction, and said peptide is at least 40% homologous to the LPI protein.

2. Peptide or polypeptide as claimed in claim 1,
wherein the part of the nucleotide sequence as defined in claim 1 under a) corresponds to nucleotides 1 to 490 of **Figure 2a (SEQ ID NO:2).**

3. Peptide or polypeptide as claimed in claim 1,
wherein the part of the nucleotide sequence as defined in claim 1 under a) corresponds to nucleotides 41 to 490 of **Figure 2a (SEQ ID NO:2).**

4. Peptide or polypeptide as claimed in claim 1,
wherein the part of the nucleotide sequence as defined in claim 1 under a) corresponds to nucleotides 125 to 490 of **Figure 2a (SEQ ID NO:2).**

5. Peptide or polypeptide as claimed in claim 1,
wherein the party of the nucleotide sequence as defined in claim 1 under a) corresponds to nucleotides 1 to 490 of lpi-B **(SEQ ID NO:4)** or lpi-C **(SEQ ID NO:6)** in **Figure 2b.**

6. Peptide or polypeptide as claimed in claim 1,
wherein the part of the nucleotide sequence as defined in claim 1 under a) corresponds to nucleotides 41 to 490 of lpi-B **(SEQ ID NO:4)** or lpi-C **(SEQ ID NO:6)** in Figure 2b.

7. Peptide or polypeptide as claimed in claim 1,
wherein the part of the nucleotide sequence as defined in claim 1 under a) corresponds to nucleotides 125 to 490 of lpi-B **(SEQ ID NO:4)** or lpi-C **(SEQ ID NO:6)** in **Figure 2b.**

8. Peptide or polypeptide as claimed in claims 1-7,
wherein the nucleotide sequence as defined in claim 1 under d has an identity being at least 50% identical to any one of the nucleotide sequences a, b or c.

9. Peptide or polypeptide as claimed in claims 1-7,
wherein the nucleotide sequence as defined in claim 1 under d has an identity being at least 60% identical to any one of the nucleotide sequences a, b or c.

10. Peptide or polypeptide as claimed in claims 1-7,
wherein the nucleotide sequence as defined in claim 1 under d has an identity being at least 70% identical to any one of the nucleotide sequences a, b or c.

11. Peptide or polypeptide as claimed in claims 1-7,
wherein the nucleotide sequence as defined in claim 1 under d has an identity being at least 75% identical to any one of the nucleotide sequences a, b or c.

12. Peptide or polypeptide as claimed in claims 1-7,
wherein the nucleotide sequence as defined in claim 1 under d has an identity being at least 80% identical to any one of the nucleotide sequences a, b or c.

13. Peptide or polypeptide as claimed in claims 1-7,
wherein the nucleotide sequence as defined in claim 1 under d has an identity being at least 90% identical to any one of the nucleotide sequences a, b or c.

14. Peptide or polypeptide as claimed in claims 1-13,
wherein the stringent conditions are constituted by overnight hybridization at 42°C in 5xSSC and washing at 65°C at 0.1xSSC.

15. Peptide or polypeptide as claimed in claims 1-14,
wherein a portion of the amino acid sequence as defined in claim 1 under c) constitutes alone or with other portions of the amino acid sequence the region(s) of the peptide or polypeptide having LPI activity.

16. Use of the peptide or polypeptide as claimed in claims 1-15 for the manufacture of a therapeutic preparation for prophylaxis or treatment of acute and chronic inflammation reactions.

17. A therapeutic composition comprising a suitable excipient and the peptide or polypeptide as claimed in claims 1-15 for prophylaxis or treatment of acute and chronic inflammation reactions.

18. Micro-organism harboring a nucleic acid molecule as defined in claims 1-15 for use as a medicament for the treatment of acute and chronic inflammation reactions.

## Patentansprüche

1. Peptid oder Polypeptid, das von einer Nukleotidsequenz codiert wird, welche einer Sequenz entspricht, die ausgewählt ist aus der Gruppe, bestehend aus:
a) einer Nukleotidsequenz, welche einen Teil von einer der Sequenzen, wie sie in **Figur 2a** und **2b** dargestellt sind und als **SEQ ID NR:2, SEQ ID NR:4** und **SEQ** ID **NR:6** identifiziert werden, umfasst;
b) Nukleotidsequenzen, welche ein Peptid oder Polypeptid mit der Aminosäuresequenz, die in **Figur 3** dargestellt ist und als Sequenz **SEQ ID NR:3, SEQ ID NR:5** oder **SEQ ID NR:7** identifiziert wird, codieren;
c) Nukleotidsequenzen, welche ein Peptid oder Polypeptid mit einem Teil der Aminosäuresequenz, die in **Figur 3** dargestellt ist und als Sequenz **SEQ ID NR:3, SEQ ID NR:5** oder SEQ **ID NR:7** identifiziert wird, codieren;
d) Nukleotidsequenzen, die zu wenigstens 40% mit irgendeiner der Nukleotidsequenzen a), b) oder c) identisch sind;
e) Nukleotidsequenzen, die unter stringenten Bedingungen mit irgendeiner der Nukleotidsequenzen a), b), c) oder d) hybridisieren; und
f) Nukleotidsequenzen, die zu irgendeiner der Nukleotidsequenzen a), b), c), d) oder e) komplementär sind,
wobei das Peptid oder Polypeptid LPI (Inhibitor des Lectinwegs)-Aktivität aufweist, was eine Aktivierung des Lectinwegs der Komplementaktivierung verhindert, indem spezifisch die von MASP-2 (Mannose bindende Lectin-assoziierte Serinprotease-2) abhängige Spaltung von C2 zu C2a und C2b verhindert wird, und das Peptid oder Polypeptid zur Verwendung bei der Prophylaxe oder Behandlung einer akuten und chronischen Entzündungsreaktion dient und das Peptid zu wenigstens 40% zu dem LPI-Protein homolog ist.

2. Peptid oder Polypeptid wie in Anspruch 1 beansprucht, wobei der Teil der Nukleotidsequenz, wie sie in Anspruch 1 unter a) definiert ist, den Nukleotiden 1 bis 490 von **Figur 2a (SEQ ID NR:2)** entspricht.

3. Peptid oder Polypeptid wie in Anspruch 1 beansprucht, wobei der Teil der Nukleotidsequenz, wie sie in Anspruch 1 unter a) definiert ist, den Nukleotiden 41 bis 490 von **Figur 2a (SEQ ID NR:2)** entspricht.

4. Peptid oder Polypeptid wie in Anspruch 1 beansprucht, wobei der Teil der Nukleotidsequenz, wie sie in Anspruch 1 unter a) definiert ist, den Nukleotiden 125 bis 490 von **Figur** 2a **(SEQ ID NR:2)** entspricht.

5. Peptid oder Polypeptid wie in Anspruch 1 beansprucht, wobei der Teil der Nukleotidsequenz, wie sie in Anspruch 1 unter a) definiert ist, den Nukleotiden 1 bis 490 von lpi-B **(SEQ ID NR:4)** oder lpi-C **(SEQ ID NR:6)** in **Figur 2b** entspricht.

6. Peptid oder Polypeptid wie in Anspruch 1 beansprucht, wobei der Teil der Nukleotidsequenz, wie sie in Anspruch 1 unter a) definiert ist, den Nukleotiden 41 bis 490 von lpi-B **(SEQ ID NR:4)** oder lpi-C **(SEQ ID NR:6)** in **Figur 2b** entspricht.

7. Peptid oder Polypeptid wie in Anspruch 1 beansprucht, wobei der Teil der Nukleotidsequenz, wie sie in Anspruch 1 unter a) definiert ist, den Nukleotiden 125 bis 490 von lpi-B **(SEQ ID NR:4)** oder 1pi-C **(SEQ ID NR:6)** in Figur **2b** entspricht.

8. Peptid oder Polypeptid wie in den Ansprüchen 1-7 beansprucht, wobei die Nukleotidsequenz, wie sie in Anspruch 1 unter d definiert ist, eine Identität aufweist, die wenigstens zu 50% mit irgendeiner der Nukleotidsequenzen a, b oder c identisch ist.

9. Peptid oder Polypeptid wie in den Ansprüchen 1 - 7 beansprucht, wobei die Nukleotidsequenz, wie sie in Anspruch 1 unter d definiert ist, eine Identität aufweist, die wenigstens zu 60% mit irgendeiner der Nukleotidsequenzen a, b oder c identisch ist.

10. Peptid oder Polypeptid wie in den Ansprüchen 1 - 7 beansprucht, wobei die Nukleotidsequenz, wie sie in Anspruch 1 unter d definiert ist, eine Identität aufweist, die wenigstens zu 70% mit irgendeiner der Nukleotidsequenzen a, b oder c identisch ist.

11. Peptid oder Polypeptid wie in den Ansprüchen 1-7 beansprucht, wobei die Nukleotidsequenz, wie sie in Anspruch 1 unter d definiert ist, eine Identität aufweist, die wenigstens zu 75% mit irgendeiner der Nukleotidsequenzen a, b oder c identisch ist.

12. Peptid oder Polypeptid wie in den Ansprüchen 1 - 7 beansprucht, wobei die Nukleotidsequenz, wie sie in Anspruch 1 unter d definiert ist, eine Identität aufweist, die wenigstens zu 80% mit irgendeiner der Nukleotidsequenzen a, b oder c identisch ist.

13. Peptid oder Polypeptid wie in den Ansprüchen 1 - 7 beansprucht, wobei die Nukleotidsequenz, wie sie in Anspruch 1 unter d definiert ist, eine Identität aufweist, die wenigstens zu 90% mit irgendeiner der Nukleotidsequenzen a, b oder c identisch ist.

14. Peptid oder Polypeptid wie in den Ansprüchen 1-13 beansprucht, wobei die stringenten Bedingungen durch eine Hybridisierung über Nacht bei 42°C in 5x SSC und ein Waschen bei 65°C bei 0,1x SSC gebildet werden.

15. Peptid oder Polypeptid wie in den Ansprüchen 1-14 beansprucht, wobei ein Teil der Aminosäuresequenz, wie sie in Anspruch 1 unter c) definiert wird, allein oder mit anderen Teilen der Aminosäuresequenz den Bereich (die Bereiche) des Peptids oder Polypeptids mit LPI-Aktivität bildet.

16. Verwendung des Peptids oder Polypeptids wie in den Ansprüchen 1-15 beansprucht zur Herstellung eines therapeutischen Präparats zur Prophylaxe oder Behandlung von akuten und chronischen Entzündungsreaktionen.

17. Eine therapeutische Zusammensetzung, welche einen geeigneten Arzneimittelträger und das Peptid oder Polypeptid wie in den Ansprüchen 1-15 beansprucht umfasst, zur Prophylaxe oder Behandlung von akuten und chronischen Entzündungsreaktionen.

18. Mikroorganismus, welcher ein Nukleinsäuremolekül wie in den Ansprüchen 1-15 definiert beherbergt, zur Verwendung als ein Medikament zur Behandlung von akuten und chronischen Entzündungsreaktionen.

## Revendications

1. Peptide ou polypeptide codé par une séquence nucléotidique correspondant à une séquence qui est sélectionnée parmi le groupe consistant en :
a) une séquence nucléotidique comprenant une partie d'une des séquences telles que décrites dans la figure 2a et 2b et identifiées en tant que SEQ ID NO: 2 ; SEQ ID NO: 4 ; SEQ ID NO: 6 ;
b) des séquences nucléotidiques codant pour un peptide ou un polypeptide ayant la séquence d'acides aminés décrite dans la figure 3 et identifiées en tant que SEQ ID NO: 3 ; SEQ ID NO: 5 ou SEQ ID NO: 7 ;
c) des séquences nucléotidiques codant pour un peptide ou un polypeptide ayant une partie de la séquence d'acides aminés décrite dans la figure 3 identifiées en tant que SEQ ID NO: 3 ; SEQ ID NO: 5 ou SEQ ID NO: 7 ;
d) des séquences nucléotidiques qui sont au moins à 40 % identiques à l'une quelconque des séquences nucléotidiques a), b) ou c) ;
e) des séquences nucléotidiques s'hybridant dans des conditions de stringence avec l'une quelconque des séquences nucléotidiques a), b), c) ou d),
f) des séquences nucléotidiques complémentaires à l'une quelconque des séquences nucléotidiques a), b), c), d) ou e),
où ledit peptide ou polypeptide présente une activité d'inhibiteur de la voie des lectines (LPI) qui empêche l'activation de la voie des lectines de l'activation du complément en empêchant spécifiquement le clivage dépendant de la MASP-2 (sérine protéase-2 associée aux lectines se liant au mannose) de C2 en C2a et C2b, et ledit peptide ou polypeptide est destiné à une utilisation pour la prévention ou le traitement d'une réaction inflammatoire aiguë et chronique, et ledit peptide est au moins à 40 % homologue de la protéine LPI.

2. Peptide ou polypeptide selon la revendication 1, où la partie de la séquence nucléotidique telle que définie dans la revendication 1 selon a) correspond aux nucléotides 1 à 490 de la figure 2a (SEQ ID NO: 2).

3. Peptide ou polypeptide selon la revendication 1, où la partie de la séquence nucléotidique telle que définie dans la revendication 1 selon a) correspond aux nucléotides 41 à 490 de la figure 2a (SEQ ID NO: 2).

4. Peptide ou polypeptide selon la revendication 1, où la partie de la séquence nucléotidique telle que définie dans la revendication 1 selon a) correspond aux nucléotides 125 à 490 de la figure 2a (SEQ ID NO: 2).

5. Peptide ou polypeptide selon la revendication 1, où la partie de la séquence nucléotidique telle que définie dans la revendication 1 selon a) correspond aux nucléotides 1 à 490 de lpi-B (SEQ ID NO:4) ou lpi-C (SEQ ID NO: 6) dans la figure 2b.

6. Peptide ou polypeptide selon la revendication 1, où la partie de la séquence nucléotidique telle que définie dans la revendication 1 selon a) correspond aux nucléotides 41 à 490 de 1pi-B (SEQ ID NO:4) ou lpi-C (SEQ ID NO: 6) dans la figure 2b.

7. Peptide ou polypeptide selon la revendication 1, où la partie de la séquence nucléotidique telle que définie dans la revendication 1 selon a) correspond aux nucléotides 125 à 490 de lpi-B (SEQ ID NO:4) ou lpi-C (SEQ ID NO: 6) dans la figure 2b.

8. Peptide ou polypeptide selon les revendications 1 à 7, où la séquence nucléotidique telle que définie dans la revendication 1 selon d possède une identité qui est au moins à 50 % identique à l'une quelconque des séquences nucléotidiques a, b ou c.

9. Peptide ou polypeptide selon les revendications 1 à 7, où la séquence nucléotidique telle que définie dans la revendication 1 selon d possède une identité qui est au moins à 60 % identique à l'une quelconque des séquences nucléotidiques a, b ou c.

10. Peptide ou polypeptide selon les revendications 1 à 7, où la séquence nucléotidique telle que définie dans la revendication 1 selon d possède une identité qui est au moins à 70 % identique à l'une quelconque des séquences nucléotidiques a, b ou c.

11. Peptide ou polypeptide selon les revendications 1 à 7, où la séquence nucléotidique telle que définie dans la revendication 1 selon d possède une identité qui est au moins à 75 % identique à l'une quelconque des séquences nucléotidiques a, b ou c.

12. Peptide ou polypeptide selon les revendications 1 à 7, où la séquence nucléotidique telle que définie dans la revendication 1 selon d possède une identité qui est au moins à 80 % identique à l'une quelconque des séquences nucléotidiques a, b ou c.

13. Peptide ou polypeptide selon les revendications 1 à 7, où la séquence nucléotidique telle que définie dans la revendication 1 selon d possède une identité qui est au moins à 90 % identique à l'une quelconque des séquences nucléotidiques a, b ou c.

14. Peptide ou polypeptide selon les revendications 1 à 13, où les conditions de stringence sont constituées par une hybridation toute une nuit à 40°C dans 5 x SSC et un lavage à 65°C à 0,1 x SSC.

15. Peptide ou polypeptide selon les revendications 1 à 14, où une partie de la séquence d'acides aminés telle que définie dans la revendication 1 selon c) constitue seule ou avec d'autres parties de la séquence d'acides aminés la ou les région(s) du peptide ou polypeptide ayant une activité LPI.

16. Utilisation du peptide ou polypeptide selon les revendications 1 à 15 pour la fabrication d'une préparation thérapeutique pour la prévention ou le traitement de réactions inflammatoires aiguës et chroniques.

17. Composition thérapeutique comprenant un excipient approprié et le peptide ou polypeptide selon les revendications 1 à 15 pour la prévention ou le traitement de réactions inflammatoires aiguës et chroniques.

18. Micro-organisme hébergeant une molécule d'acide nucléique tel que défini dans les revendications 1 à 15 pour une utilisation en tant qu'un médicament pour le traitement de réactions inflammatoires aiguës et chroniques.
